# EUROPEAN PATENT APPLICATION

(11) **EP 3 913 002 A1**
(43) Date of publication of application: **24.11.2021**
(21) Application number: 19879120.4
(22) Date of filing: 28.08.2019
(51) Int. Cl.: C07K 19/00, C12N 15/62, C12N 5/10, A61K 38/17

(54) **APPLICATION OF ECM1 IN PREVENTION AND/OR TREATMENT OF LIVER FIBROSIS-RELATED DISEASES**

(30) Priority: 02.11.2018 CN 201811301573
(71) Applicant: Center for Excellence in Molecular Cell Science, Chinese Academy of Sciences, Shanghai 200031 (CN)
(72) Inventor: SUN, Bing, Shanghai 200031 (CN); FAN, Weiguo, Shanghai 200031 (CN); ZHANG, Yaguang, Shanghai 200031 (CN); LING, Zhiyang, Shanghai 200031 (CN)
(74) Representative: Rondano, Davide
(86) International application number: PCT/CN2019/103127
(87) International publication number: WO 2020/088070

(57) **Abstract**

Provided is application of ECM1 in the prevention and/or treatment of liver fibrosis-related diseases, specifically provided is the use of ECM1 gene, or protein or a promoter thereof for preparing a composition or a formulation, the composition or formulation being used for (a) preventing and/or treating of liver fibrosis-related diseases; and/or for (b) maintaining liver homeostasis. The ECM1 gene, or the protein or promoter thereof can significantly (i) prevent and/or treat cirrhosis-related diseases; and/or (ii) maintain the liver homeostasis. In addition, the ECM1 gene, or the protein or promoter thereof can also significantly (i) inhibit the occurrence of liver fibrosis-related diseases; and/or (ii) inhibit the activation of hepatic stellate cells (HSCs).

Sequence listing

<110>  CENTER FOR EXCELLENCE IN MOLECULAR CELL SCIENCE, CHINESE ACADEMY OF
SCIENCES

<120>  APPLICATION OF ECM1 IN PREVENTION AND/OR TREATMENT OF LIVER
FIBROSIS-RELATED DISEASES

<130>  P2019-1252

<150>  CN201811301573.2
<151>  2018-11-02

<160>  4

<170>  PatentIn version 3.5

<210>  1
<211>  559
<212>  PRT
<213>  Mus musculus

<400>  1

Met Gly Thr Val Ser Arg Ala Ala Leu Ile Leu Ala Cys Leu Ala Leu
1               5                   10                  15

Ala Ser Ala Ala Ser Glu Gly Ala Phe Lys Ala Ser Asp Gln Arg Glu
            20                  25                  30

Met Thr Pro Glu Arg Leu Phe Gln His Leu His Glu Val Gly Tyr Ala
        35                  40                  45

Ala Pro Pro Ser Pro Pro Gln Thr Arg Arg Leu Arg Val Asp His Ser
    50                  55                  60

Val Thr Ser Leu His Asp Pro Pro Leu Phe Glu Glu Gln Arg Glu Val
65                  70                  75                  80

Gln Pro Pro Ser Ser Pro Glu Asp Ile Pro Val Tyr Glu Glu Asp Trp
                85                  90                  95

Pro Thr Phe Leu Asn Pro Asn Val Asp Lys Ala Gly Pro Ala Val Pro
            100                 105                 110

Gln Glu Ala Ile Pro Leu Gln Lys Glu Gln Pro Pro Pro Gln Val His
        115                 120                 125

Ile Glu Gln Lys Glu Ile Asp Pro Pro Ala Gln Pro Gln Glu Glu Ile
    130                 135                 140

Val Gln Lys Glu Val Lys Pro His Thr Leu Ala Gly Gln Leu Pro Pro
145                 150                 155                 160

Glu Pro Arg Thr Trp Asn Pro Ala Arg His Cys Gln Gln Gly Arg Arg
                165                 170                 175

Gly Val Trp Gly His Arg Leu Asp Gly Phe Pro Pro Gly Arg Pro Ser
            180                 185                 190

Pro Asp Asn Leu Lys Gln Ile Cys Leu Pro Glu Arg Gln His Val Ile
        195                 200                 205

Tyr Gly Pro Trp Asn Leu Pro Gln Thr Gly Tyr Ser His Leu Ser Arg
    210                 215                 220

Gln Gly Glu Thr Leu Asn Val Leu Glu Thr Gly Tyr Ser Arg Cys Cys
225                 230                 235                 240

Arg Cys Arg Ser Asp Thr Asn Arg Leu Asp Cys Leu Lys Leu Val Trp
                245                 250                 255

Glu Asp Ala Met Thr Gln Phe Cys Glu Ala Glu Phe Ser Val Lys Thr
            260                 265                 270

Arg Pro His Leu Cys Cys Arg Leu Arg Gly Glu Glu Arg Phe Ser Cys
        275                 280                 285

Phe Gln Lys Glu Ala Pro Arg Pro Asp Tyr Leu Leu Arg Pro Cys Pro
    290                 295                 300

Val His Gln Asn Gly Met Ser Ser Gly Pro Gln Leu Pro Phe Pro Pro
305                 310                 315                 320

Gly Leu Pro Thr Pro Asp Asn Val Lys Asn Ile Cys Leu Leu Arg Arg
                325                 330                 335

Phe Arg Ala Val Pro Arg Asn Leu Pro Ala Thr Asp Ala Ile Gln Arg
            340                 345                 350

Gln Leu Gln Ala Leu Thr Arg Leu Glu Thr Glu Phe Gln Arg Cys Cys
        355                 360                 365

Arg Gln Gly His Asn His Thr Cys Thr Trp Lys Ala Trp Glu Gly Thr
    370                 375                 380

Leu Asp Gly Tyr Cys Glu Arg Glu Leu Ala Ile Lys Thr His Pro His
385                 390                 395                 400

Ser Cys Cys His Tyr Pro Pro Ser Pro Ala Arg Asp Glu Cys Phe Ala
                405                 410                 415

His Leu Ala Pro Tyr Pro Asn Tyr Asp Arg Asp Ile Leu Thr Leu Asp
                420                 425                 430

Leu Ser Arg Val Thr Pro Asn Leu Met Gly Gln Leu Cys Gly Ser Gly
        435                 440                 445

Arg Val Leu Ser Lys His Lys Gln Ile Pro Gly Leu Ile Gln Asn Met
    450                 455                 460

Thr Ile Arg Cys Cys Glu Leu Pro Tyr Pro Glu Gln Ala Cys Cys Gly
465                 470                 475                 480

Glu Glu Glu Lys Leu Ala Phe Ile Glu Asn Leu Cys Gly Pro Arg Arg
                485                 490                 495

Asn Ser Trp Lys Asp Pro Ala Leu Cys Cys Asp Leu Ser Pro Glu Asp
                500                 505                 510

Lys Gln Ile Asn Cys Phe Asn Thr Asn Tyr Leu Arg Asn Val Ala Leu
        515                 520                 525

Val Ala Gly Asp Thr Gly Asn Ala Thr Gly Leu Gly Glu Gln Gly Pro
    530                 535                 540

Thr Arg Gly Thr Asp Ala Asn Pro Ala Pro Gly Ser Lys Glu Glu
545                 550                 555

<210>  2
<211>  1680
<212>  DNA
<213>  Mus musculus

<400>  2
atggggaccg tatccagagc agccttgatc ttggcctgct tggctcttgc ttctgctgcc      60

tctgagggag ccttcaaggc ttcagaccag cgagagatga cgccagagcg cctcttccag     120

cacctccatg aagtaggtta tgcagcaccc ccttccccac cacaaacccg gagactccga     180

gttgaccact ctgtaacttc tctgcatgac cctcccctct ttgaggaaca aagagaagtg     240

cagccccctt cctctccaga agacatccct gtgtacgagg aagactggcc cactttccta     300

aaccctaatg tagataaagc tggtcctgct gtccctcaag aagccatccc cctgcagaaa     360

gagcagcccc ctccccaagt ccatattgaa cagaaggaaa tagacccgcc tgcccagcct     420

caggaggaga ttgtccagaa agaggtgaag ccacacacct tggcggccca gctccctcca     480

gagccccgga cttggaatcc agcccgtcac tgccagcagg gacggagagg tgtctggggc     540

caccggctgg atggcttccc tcctggacgg ccttctccag acaatctgaa gcagatctgc     600

cttcctgagc gtcagcatgt gatctacggc ccctggaacc tgccgcagac tggctactct 660

caccttagtc gccagggaga gaccctcaat gtgctggaga ccggatactc ccgctgctgt 720

cgctgccgca gcgacacaaa ccgcctagac tgtttgaagc ttgtgtggga ggatgcaatg 780

acccaatttt gtgaggccga attctctgtc aagacccgcc cccacctgtg ctgcagactg 840

cgtggggagg agcgattctc ttgcttccag aaggaagctc ctcgcccaga ctacctgctc 900

cgaccctgcc ccgtccacca gaatggcatg tcctcagggc cccagttgcc tttccccccg 960

gggttgccca caccggacaa tgtcaaaaac atctgtctcc tgagacgctt ccgcgccgtg 1020

ccacgcaacc tcccagctac tgacgccatc cagaggcagc tgcaggctct gactcggctg 1080

gagacggagt tccagcgctg ctgccgccag ggccacaacc acacttgcac atggaaggcc 1140

tgggagggta ccctggatgg atactgcgag cgggagctgg ctataaagac ccacccccac 1200

tcgtgctgcc actaccctcc tagtcctgcc cgtgatgagt gcttcgccca cctagctccc 1260

tatcccaact atgaccggga tatcttgacc cttgacctca gccgagtcac ccccaacctc 1320

atgggccagc tctgtggaag tggaagggtc cttagcaagc ataaacagat tccggggctg 1380

atccagaata tgaccatccg ctgctgcgag cttccatatc cagaacaggc ctgctgcggc 1440

gaagaggaga aactggcctt catcgagaac ctctgtggtc cccggaggaa ttcgtggaaa 1500

gaccctgccc tctgctgtga cctgtctcct gaagataagc aaatcaactg cttcaatacc 1560

aactacctga ggaacgtggc tttagtggct ggagacactg ggaatgccac tggcttgggg 1620

gagcagggcc caactcgggg aacagatgcc aaccccgccc ctgggtccaa ggaagaatga 1680

<210> 3
<211> 540
<212> PRT
<213> Homo sapiens

<400> 3

<210>  4
<211>  1623
<212>  DNA
<213>  Homo sapiens

<400>  4
atggggacca cagccagagc agccttggtc ttgacctatt tggctgttgc ttctgctgcc        60

tctgagggag gcttcacggc tacaggacag aggcagctga ggccagagca ctttcaagaa 120

gttggctacg cagctccccc ctccccaccc ctatcccgaa gcctccccat ggatcaccct 180

gactcctctc agcatggccc tccctttgag ggacagagtc aagtgcagcc ccctccctct 240

caggaggcca cccctctcca acaggaaaag ctgctacctg cccaactccc tgctgaaaag 300

gaagtgggtc cccctctccc tcaggaagct gtccccctcc aaaaagagct gccctctctc 360

cagcacccca atgaacagaa ggaaggaacg ccagctccat ttggggacca gagccatcca 420

gaacctgagt cctggaatgc agcccagcac tgccaacagg accggtccca agggggctgg 480

ggccaccggc tggatggctt cccccctggg cggccttctc cagacaatct gaaccaaatc 540

tgccttccta accgtcagca tgtggtatat ggtccctgga acctaccaca gtccagctac 600

tcccacctca ctcgccaggg tgagaccctc aatttcctgg agattggata ttcccgctgc 660

tgccactgcc gcagccacac aaaccgccta gagtgtgcca aacttgtgtg ggaggaagca 720

atgagccgat tctgtgaggc cgagttctcg gtcaagaccc gaccccactg gtgctgcacg 780

cggcaggggg aggctcggtt ctcctgcttc caggaggaag ctccccagcc acactaccag 840

ctccgggcct gccccagcca tcagcctgat atttcctcgg gtcttgagct gcctttccct 900

cctggggtgc ccacattgga caatatcaag aacatctgcc acctgaggcg cttccgctct 960

gtgccacgca acctgccagc tactgacccc ctacaaaggg agctgctggc actgatccag 1020

ctggagaggg agttccagcg ctgctgccgc caggggaaca atcacacctg tacatggaag 1080

gcctgggagg atacccttga caaatactgt gaccgggagt atgctgtgaa gacccaccac 1140

cacttgtgtt gccgccaccc tcccagccct actcgggatg agtgctttgc ccgtcgggct 1200

ccttacccca actatgaccg ggacatcttg accattgaca tcggtcgagt cacccccaac 1260

ctcatgggcc acctctgtgg aaaccaaaga gttctcacca agcataaaca tattcctggg 1320

ctgatccaca acatgactgc ccgctgctgt gacctgccat ttccagaaca ggcctgctgt 1380

gcagaggagg agaaattaac cttcatcaat gatctgtgtg gtccccgacg taacatctgg 1440

cgagaccctg ccctctgctg ttacctgagt cctggggatg aacaggtcaa ctgcttcaac 1500

atcaattatc tgaggaacgt ggctctagtg tctggagaca ctgagaacgc caagggccag 1560

ggggagcagg gctcaactgg aggaacaaat atcagctcca cctctgagcc caaggaagaa 1620

tga 1623

## Description

### Technical field

The present invention relates to the field of biomedicine. Specifically, the present invention relates to a use of ECM1 in the prevention and/or treatment of a liver fibrosis-related disease.

### Background

Liver fibrosis is a dynamic repair process of liver damage, similar to wound repair reaction, which mainly manifests the abnormal proliferation of collagen in the liver and excessive deposition of extracellular matrix. It is an early stage of the disease that develops to liver cirrhosis and liver cancer. Any factor that causes long-term, chronic damage to the liver will induce this repair response. China is a major liver disease country. About 100 million people are chronically infected by Hepatitis B virus, and 15 million people are chronically infected by Hepatitis C virus. At the same time, a large number of patients suffer from liver fibrosis and liver cirrhosis caused by alcoholic liver and non-alcoholic fatty liver disease. The occurrence of liver fibrosis may not only lead to liver cancer, but also a series of diseases such as portal hypertension, ascites, and encephalopathy. Although liver fibrosis has a huge impact, the research on liver fibrosis has been proceeding slowly.

The liver is mainly composed of hepatocytes, hepatic stellate cells (HSC), hepatic macrophages (Kupffer) and liver sinusoidal endothelial cells (LSEC). The extracellular matrix (ECM) of the liver not only provides a scaffold for these cells to form the liver, but also plays an important role in maintaining the physiological homeostasis of the liver. It is an intermediary of the signal communication between different cells in the liver, and it is also a reservoir of various cytokines, regulating the functions of different cells in the liver. Under pathological conditions, excessive deposition of extracellular matrix in the liver leads to liver fibrosis. The study of the function of extracellular matrix proteins can give us an in-depth understanding of how the extracellular matrix maintains a steady state under physiological conditions and inhibits the occurrence of fibrosis.

However, currently no effective drugs for the treatment of liver fibrosis have entered the clinic, and no drugs have been officially approved for clinical use in the treatment of liver fibrosis.

Therefore, there is an urgent need in this field to in-depth study the pathogenesis of liver fibrosis and explore effective drugs for preventing and/or treating liver fibrosis-related diseases.

### Summary of the invention

The purpose of the present invention is to provide an effective medicament for preventing and/or treating liver fibrosis-related diseases.

In a first aspect of the present invention, it provides a use of *ECM1* gene, or a protein thereof or a promoter thereof for the preparation of a composition or a preparation for (a) preventing and/or treating a liver fibrosis-related disease; and/or (b) maintaining liver homeostasis.

In another preferred embodiment, the composition or preparation is also used to (i) inhibit the occurrence of a liver fibrosis-related disease; and/or (ii) inhibit the activation of a hepatic stellate cell (HSC).

In another preferred embodiment, the hepatic stellate cell is the hepatic stellate cell in the sinus hepaticus.

In another preferred embodiment, the promoter refers to a substance that can increase the activity and/or content of the ECM1 gene or a protein thereof in vivo or in vitro; the substance can be a synthetic or natural compound, protein, or nucleotide etc.

In another preferred embodiment, the ECM1 promoter includes a substance that promotes the expression of ECM1.

In another preferred embodiment, the ECM1 promoter includes a substance that promotes the formation of a complex between ECM1 protein and integrin αv.

In another preferred embodiment, the ECM1 promoter includes an ECM1 protein promoter and/or an ECM1 gene promoter.

In another preferred embodiment, the promotion of ECM1 expression or activity refers to increasing the expression or activity of ECM1 gene or protein by ≥20%, preferably, ≥50%, more preferably, ≥70%.

In another preferred embodiment, the ECM1 promoter is selected from the group consisting of a small molecule compound, a vector expressing ECM1, and a combination thereof.

In another preferred embodiment, the vector for expressing ECM1 includes a viral vector.

In another preferred embodiment, the viral vector is selected from the group consisting of: an adeno-associated virus vector, lentiviral vector, and a combination thereof.

In another preferred embodiment, the protein includes a full-length protein or protein fragment.

In another preferred embodiment, the *ECM1* gene or the protein thereof is derived from a mammal, more preferably from a rodent (such as a mouse, rat), primate and human.

In another preferred embodiment, the ECM1 protein also includes a derivative of ECM1 protein.

In another preferred embodiment, the derivative of the ECM1 protein includes a modified ECM1 protein, a protein molecule whose amino acid sequence is homologous to the natural ECM1 protein and has natural ECM1 protein activity, a dimer or multimer of the ECM1 protein, a fusion protein containing the amino acid sequence of the ECM1 protein.

In another preferred embodiment, the modified ECM1 protein is a PEGylated ECM1 protein.

In another preferred embodiment, the "protein molecule whose amino acid sequence is homologous to the natural ECM1 protein and has natural ECM1 protein activity" means that a protein molecule whose amino acid sequence has ≥85% homology, preferably ≥90% homology, more preferably ≥95% homology, and most preferably ≥98% homology with ECM1 protein and has natural ECM1 protein activity.

In another preferred embodiment, the ECM1 protein is selected from the group consisting of:
(A) A polypeptide whose amino acid sequence is shown in SEQ ID NO.: 1 or 3;
(B) a ECM1 protein derivative or an active fragment thereof formed by substitution, deletion or addition of one or several (usually 1-60, preferably 1-30, more preferably 1-20, most preferably 1-10) amino acid residues in the amino acid sequence as shown in SEQ ID NO.: 1 or 3;
(C) compared with the amino acid sequence as shown in SEQ ID NO.: 1 or 3, a ECM1 protein derivative or active fragment thereof with homology ≥90%, preferably ≥95%, more preferably ≥98%, and most preferably ≥99%.

In another preferred embodiment, the ECM1 gene encodes the ECM1 protein.

In another preferred embodiment, the ECM1 gene is selected from the group consisting of:
(a) a polynucleotide encoding the polypeptide as shown in SEQ ID NO.: 1 or 3;
(b) a polynucleotide whose sequence is shown in SEQ ID NO.: 2 or 4;
(c) a polynucleotide whose nucleotide sequence has a homology of ≥95% (preferably ≥98%) with the sequence as shown in SEQ ID NO.: 2 or 4 and encoding the polypeptide as shown in SEQ ID NO.: 1 or 3;
(d) a polynucleotide complementary to any of the polynucleotides as described in (a) to (c).

In another preferred embodiment, the composition includes a pharmaceutical composition.

In another preferred embodiment, the pharmaceutical composition contains (a) *ECM1* gene, or a protein thereof or a promoter thereof; and (b) a pharmaceutically acceptable carrier.

In another preferred embodiment, the pharmaceutical composition is liquid, solid, or semi-solid.

In another preferred embodiment, the dosage form of the pharmaceutical composition includes a tablet, granule, capsule, oral liquid, or injection.

In another preferred embodiment, in the pharmaceutical composition, the component (a) accounts for 1-99wt% of the total weight of the pharmaceutical composition, preferably 10-90wt%, more preferably 30-70wt%.

In another preferred embodiment, the composition further includes an additional component for (a) preventing and/or treating a liver fibrosis-related disease; and/or (b) maintaining liver homeostasis.

In another preferred embodiment, the composition further includes an additional component that inhibits the activation of stellate cells (HSC).

In another preferred embodiment, the composition further includes an additional component selected from the group consisting of CWHM 12, c8, PF-573228, EMD527040, and a combination thereof.

In another preferred embodiment, the composition or preparation can be used alone or in combination in the application of (a) preventing and/or treating a liver fibrosis-related disease; and/or (b) maintaining liver homeostasis.

In another preferred embodiment, the use in combination includes: using in combination with other drugs for (a) preventing and/or treating a liver fibrosis-related disease; and/or (b) maintaining liver homeostasis.

In another preferred embodiment, the other drugs for (a) preventing and/or treating a liver fibrosis-related disease; and/or (b) maintaining liver homeostasis are selected from the group consisting of: CWHM 12, c8, PF-573228, EMD527040, and a combination thereof.

In another preferred embodiment, the liver fibrosis-related disease is selected from the group consisting of liver fibrosis, liver cirrhosis, alcoholic liver, fatty liver, autoimmune liver disease, drug-induced liver injury, viral hepatitis, and a combination thereof.

In another preferred embodiment, the patient with the liver fibrosis-related disease is simultaneously accompanied by a decrease in serum, plasma or liver ECM1 level.

In another preferred embodiment, the expression and/or regulation of the integrin-related gene in the patient with the liver fibrosis-related disease is not abnormal.

In another preferred embodiment, the composition or preparation is used for people with normal expression of the integrin-related gene.

In another preferred embodiment, the integrin-related gene is selected from the group consisting of integrin αv, integrin β1, integrin β3, integrin β5, integrin β6, integrin β8, integrin α3, integrin α5, integrin α8, integrin β1, and a combination thereof.

In a second aspect of the present invention, it provides a pharmaceutical composition comprising:
(a1) a first active ingredient for preventing and/or treating a liver fibrosis-related disease, the first active ingredient comprises: ECM1 gene, or a protein thereof or a promoter thereof;
(a2) a second active ingredient for preventing and/or treating a liver fibrosis-related disease, the second active ingredient comprises: other drugs for preventing and/or treating a liver fibrosis-related disease; and
(b) a pharmaceutically acceptable carrier.

In another preferred embodiment, in the pharmaceutical composition, the component (a1) accounts for 1-99wt% of the total weight of the pharmaceutical composition, preferably 10-90wt%, more preferably 30-70wt%.

In another preferred embodiment, in the pharmaceutical composition, the component (a2) accounts for 1-99wt% of the total weight of the pharmaceutical composition, preferably 10-90wt%, more preferably 30-70wt%.

In another preferred embodiment, the weight ratio of the first active ingredient and the second active ingredient is 1:100 to 100:1, preferably 1:10 to 10:1.

In another preferred embodiment, the pharmaceutical composition further includes an additional component for maintaining liver homeostasis.

In another preferred embodiment, the pharmaceutical composition further includes an additional component that inhibits the activation of stellate cells (HSC).

In another preferred embodiment, the other drugs for preventing and/or treating a liver fibrosis-related disease are selected from the group consisting of:
CWHM 12, c8, PF-573228, EMD527040, and a combination thereof.

In another preferred embodiment, the composition further includes an additional component selected from the group consisting of CWHM 12, c8, PF-573228, EMD527040, and a combination thereof.

In another preferred embodiment, the pharmaceutical composition can be a single compound or a mixture of multiple compounds.

In another preferred embodiment, the pharmaceutical composition is used to prepare drugs or preparations for treating or preventing a liver fibrosis-related disease.

In another preferred embodiment, the dosage form is oral administration or non-oral administration dosage form.

In another preferred embodiment, the oral administration dosage form is a tablet, powder, granule or capsule, or emulsion or syrup.

In another preferred embodiment, the non-oral administration dosage form is injection.

In another preferred embodiment, the total content of the active ingredient (a1) and the active ingredient (a2) is 1-99wt% of the total weight of the composition, more preferably 5-90wt%.

In a third aspect of the present invention, it provides a kit comprising:
(i) a first container, and the active ingredient (a1) ECM1 gene, or a protein thereof or a promoter thereof, or a drug containing the active ingredient (a) in the first container; and
(ii) a second container, and the active ingredient (a2) other drugs for preventing and/or treating a liver fibrosis-related disease, or a drug containing the active ingredient (a2) in the second container.

In another preferred embodiment, the first container and the second container are the same or different containers.

In another preferred embodiment, the drug in the first container is a single preparation containing ECM1 gene, or a protein thereof or a promoter thereof.

In another preferred embodiment, the drug in the second container is a single preparation containing other drugs for preventing and/or treating a liver fibrosis-related disease.

In another preferred embodiment, the dosage form of the drug is an oral dosage form or an injection dosage form.

In another preferred embodiment, the kit also contains instructions that describe the instructions for the combined administration of the active ingredient (a1) and the active ingredient (a2) to (i) prevent and/or treat a liver fibrosis-related disease; and /or (ii) maintain liver homeostasis.

In another preferred embodiment, the dosage forms of the preparation containing the active ingredient (a1) ECM1 gene, a protein thereof or a promoter thereof or the preparation containing other drugs for the prevention and/or treatment of a liver fibrosis-related disease are respectively including capsules, tablets, suppositories, or intravenous injections.

In another preferred embodiment, in the preparation containing the active ingredient (a1) ECM1 gene, or a protein thereof or a promoter thereof, the concentration of the ECM1 gene, or the protein thereof or the promoter thereof is 0.0001-100 mg/kg body weight , preferably 0.1-50 mg/kg body weight, more preferably 1-20 mg/kg body weight.

In a fourth aspect of the present invention, it provides a method for inhibiting a stellate cell (HSC) activation, comprising the steps:
in the presence of a ECM1 gene, or a protein thereof or a promoter thereof, culturing a stellate cell, thereby inhibiting the activation of the stellate cell (HSC).

In another preferred embodiment, the stellate cell is a stellate cell in the hepatic sinusoid.

In another preferred embodiment, the method is non-diagnostic and non-therapeutic.

In another preferred embodiment, the method is therapeutic.

In another preferred embodiment, the concentration of the ECM1 or the promoter thereof is 0.0001-100 mg/kg body weight, preferably 1-50 mg/kg body weight, more preferably 5-20 mg/kg body weight.

In a fifth aspect of the present invention, it provides a method for screening a potential therapeutic agent for a liver fibrosis-related disease, comprising:
(a) In a test group, in the culture system, in the presence of a test compound, culturing a cell expressing the *ECM1* gene for a period of time T1, and detecting the expression level E1 of the *ECM1* gene in the culture system of the test group;
   and in a control group where the test compound is absent and other conditions are the same, detecting the expression level E2 of the *ECM1* gene in the culture system of the control group; and
(b) comparing E1 and E2, if E1 is significantly higher than E2, indicating that the test compound is a potential therapeutic agent for liver fibrosis.

In another preferred embodiment, the "significantly higher" means that E1/E2>2, preferably, ≥3, more preferably, ≥4.

In another preferred embodiment, the cell includes a hepatocyte.

In another preferred embodiment, the method is non-diagnostic and non-therapeutic.

In another preferred embodiment, the method comprises step (c): administering the potential therapeutic agent determined in step (a) to a mammal, thereby determining its effect on the liver fibrosis-related disease in the mammal.

In another preferred embodiment, the mammal includes a human or non-human mammal.

In another preferred embodiment, the non-human mammal includes a rodent, primate, and preferably include a mouse, rat, rabbit, and monkey.

In a sixth aspect of the present invention, it provides a method for preventing and/or treating a liver fibrosis-related disease, comprising the steps:
administering to a subject in need a ECM1 gene, a protein thereof or a promoter thereof, the pharmaceutical composition according to the second aspect of the present invention, or the kit according to the third aspect of the present invention.

In another preferred embodiment, the administration includes oral administration.

In another preferred embodiment, the subject includes a human or non-human mammal.

In another preferred embodiment, the non-human mammal includes a rodent and primate, preferably a mouse, rat, rabbit, and monkey.

In another preferred embodiment, the dosage of administration of the ECM1 or the promoter thereof is 0.0001-100 mg/kg body weight, preferably 1-50 mg/kg body weight, and most preferably 5-20 mg/kg body weight.

In another preferred embodiment, the frequency of administration of the ECM1 or the promoter thereof is 1-150 times/month, preferably 1 day/time.

In another preferred embodiment, the administration time of the ECM1 or the promoter thereof is 5-100 days, preferably 10-50 days, and most preferably 14-42 days.

In a seventh aspect of the present invention, it provides a kit comprising:
(a1) a first container, and a therapeutic drug located in the first container, the therapeutic drug comprising a ECM1 gene, or a protein thereof, or a promoter thereof; and
(b1) a second container, and a detection reagent for an integrin-related gene in the second container.

In another preferred embodiment, the kit also contains other drugs for preventing and/or treating a liver fibrosis-related disease.

In another preferred embodiment, the integrin-related gene is selected from the group consisting of integrin αv, integrin β1, integrin β3, integrin β5, integrin β6, integrin β8, integrin α3, integrin α5, integrin α8, integrin β1, and a combination thereof.

In another preferred embodiment, the first container and the second container are the same or different containers.

In another preferred embodiment, the drug in the first container is a single preparation containing a therapeutic medicine.

In another preferred embodiment, the detection reagent is used to detect the expression of the integrin-related gene in the blood and/or liver tissue of an individual.

In another preferred embodiment, the dosage form of the drug is an oral dosage form or an injection dosage form.

In another preferred embodiment, the kit also contains instructions.

In another preferred embodiment, the individual includes an individual with no abnormality in the expression and/or regulation of the integrin-related gene in vivo.

In another preferred embodiment, the individual includes an individual who also has decreased serum, plasma, or liver ECM1 level.

In another preferred embodiment, the description records instructions selected from the group consisting of:
(a) when the expression level of the integrin-related gene in the individual's blood and/or liver tissue is equal to or higher than the predetermined standard, the ECM1 gene, or the protein thereof, or the promoter thereof is administered; wherein the predetermined standard refers to the expression level E1 of the integrin-related gene in an individual's blood and/or liver tissue / the expression level E2 of the integrin-related gene in the blood and/or liver tissue of a normal individual (E1/E2)≥50%, preferably, ≥70 %, more preferably, ≥90%,more preferably, ≥95%, more preferably, ≥120%.

In another preferred embodiment, E1/E2 is 50-200%, preferably 90-200%, more preferably 95-200%.

In an eighth aspect of the present invention, it provides a method for determining a therapeutic regimen, comprising:
a) providing a test sample from a subject;
b) detecting the expression level of the integrin-related gene in the test sample;
   and
c) determining a therapeutic regimen based on the expression level of the integrin-related gene in the sample.

In another preferred embodiment, the subject is a human or non-human mammal.

In another preferred embodiment, when the expression level of the integrin-related gene in the sample is equal to or higher than the predetermined standard, the therapeutic regimen comprises the administration of ECM1 gene, or a protein thereof, or a promoter thereof;
wherein the predetermined standard refers to the expression level E1 of the integrin-related gene in the blood and/or liver tissue of an individual / the expression level E2 of the integrin-related gene in blood and/or liver tissue of a normal individual (E1/E2) ≥50%, preferably, ≥70%, more preferably, ≥90%,more preferably, ≥95%, more preferably, ≥120%.

In another preferred embodiment, E1/E2 is 50-200%, preferably 90-200%, more preferably 95-200%.

It should be understood that, within the scope of the present invention, the technical features specifically described above and below (such as the Examples) can be combined with each other, thereby constituting a new or preferred technical solution which needs not be described one by one.

### Description of Drawings

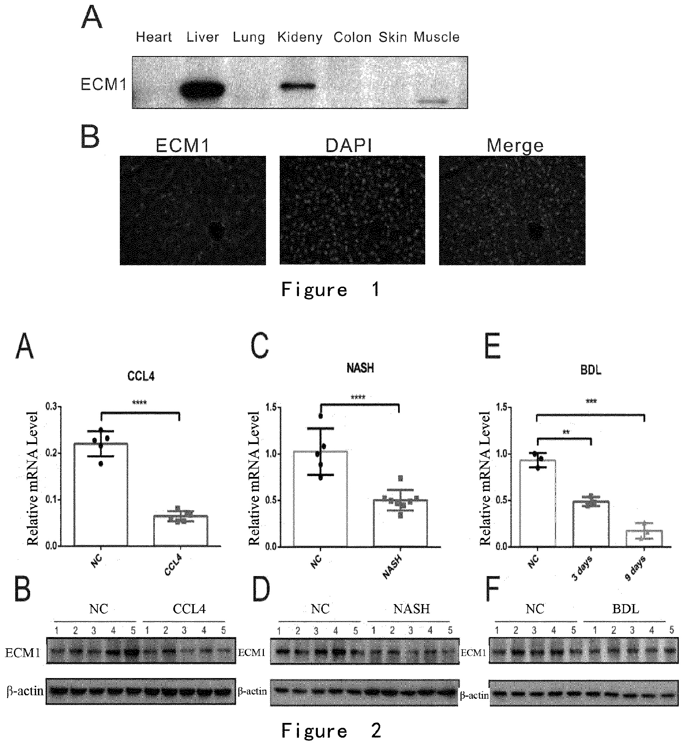

Figure 1 shows a result of ECM1 mouse tissue expression analysis.

Wherein A: Weighing 500 mg of each organ of normal mice to be lysed in 1ml RIPA lysis buffer, and subjected to grinding and pyrolysis. After quantification with BCA, uniformly diluted with RIPA lysis buffer to 2ug/ml, adding Loading Buffer for the heat denaturation. When loading the sample, each sample is 20ug/lane. Using anti-mouse ECM1 antibody to detect.

B: The liver of normal mice is fixed, dehydrated, and made into frozen sections. Using anti-mouse ECM1 antibody as a primary antibody and anti-rabbit-cy3 antibody as a secondary antibody. Taking pictures with a fluorescence microscope.

Figure 2 shows the detection results of ECM1 expression in different liver fibrosis models. Wherein

A and B: A group of normal mice are injected with lul/g body weight of CCL4 every week for 4 weeks (CCL4 group), while a group of olive oil is injected as a control (NC group). The mice are sacrificed 2 days after the last injection of CCL4, and the liver is taken for realtime and WB analysis of ECM1 expression.

C and D: A group of normal mice are fed methionine-choline deficient diet (MCD, methionine-choline deficient diet) (NASH group) for 8 weeks, while a group of normal mice are fed normal diet as a control (NC group). After 8 weeks, the liver is taken for realtime and WB analysis of ECM1 expression.

E and F: A group of normal mice are sacrificed after bile duct ligation for 3 days and their livers are taken (3 days), and a group of normal mice are sacrificed after bile duct ligation for 9 days and their livers are taken (9 days). One group of mice only underwent surgery without bile duct ligation is used as a control (NC group). Taking the liver of BDL-3 days and BDL-9 days for realtime analysis of ECM1 expression. Taking BDL-9 days liver for WB analysis of ECM1 expression.

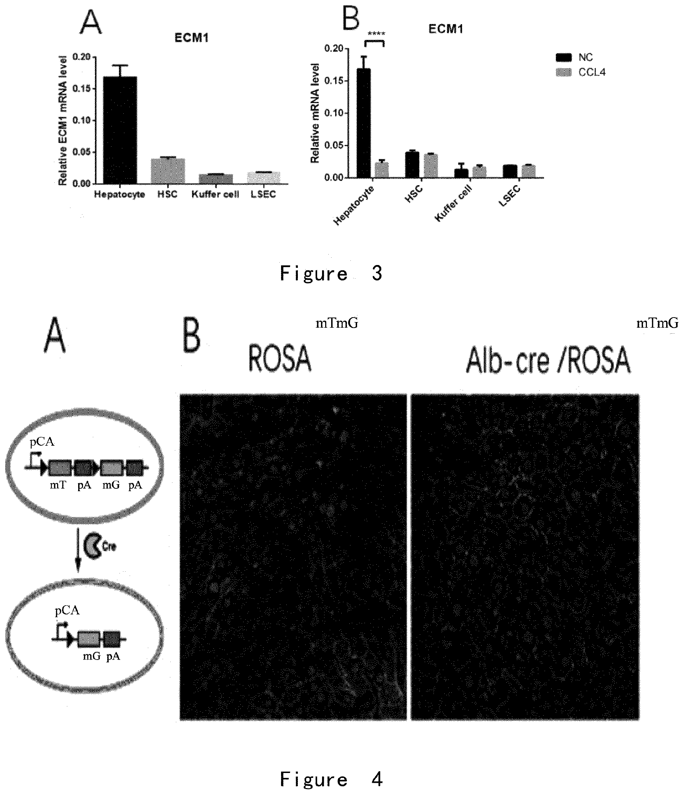

Figure 3 shows that hepatocytes are the main source of ECM1 protein in the liver. Wherein

A. Normal mouse liver is perfused in situ. After digestion, various cell suspensions are obtained by density gradient centrifugation. The highest density is Hepatocyte, HSC, Kupffer cells and LSEC, which are obtained by the respective surface characteristic molecules using magnetic beads or flow cytometry sorting. After the cells are collected, they are lysed with TRIZOL, mRNA is extracted, and cDNA is obtained by reverse transcription. Finally, the expression of ECM1 is detected by RT-PCR. GAPDH is an internal reference.

B. Four weeks after normal mice are injected with CCL4 for modeling, the liver is perfused in situ. After digestion, density gradient centrifugation is used to obtain various cell suspensions. Separation and purification of Hepatocyte, HSC, Kupffer cells and LSEC. At the same time, a group of untreated mice is used as a control. The expression of ECM1 is detected by RT-PCR. GAPDH is an internal reference.

Figure 4 shows that Alb-cre can specifically knock out genes in hepatocytes. Wherein

A. Schematic diagram of ROSA mTmG mouse fluorescent gene expression. Under normal circumstances, only the red fluorescent protein (membrane-targeted tandem dimer Tomato, mT) is expressed on the cell membrane surface in ROSA mTmG mice. The green fluorescent protein (membrane-targeted green fluorescent protein, mG) will not be transcribed because it has a stop codon in front of it. In cells with cre enzyme expression, the stop codon at the front end of the red fluorescent protein (mT) gene and the green fluorescent protein (mG) is cut, and the green fluorescent protein (mG) will replace the red fluorescent protein (mT) expressed on the cell membrane surface, turning the cell from red to green.

B. The livers of ROSA ^{mTmG} mice and Alb-cre /ROSA ^{mTmG} mice are fixed, dehydrated, and frozen sectioned, it is photographed by fluorescence microscope after dyeing DAPI-labeled nucleus.

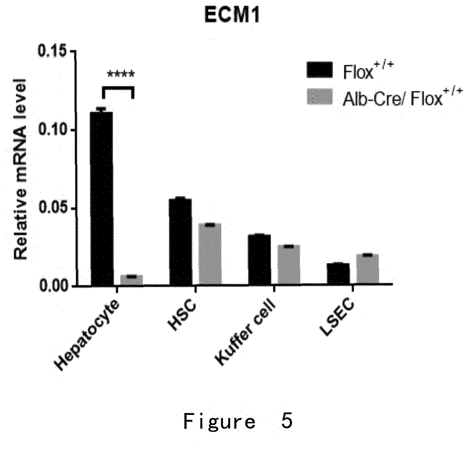

Figure 5 shows the expression verification of hepatocyte-specific knockout ECM1 gene mice. Wherein

Alb-cre /ECM1 flox^{+/+} mouse liver is perfused in situ. After digestion, various cell suspensions were obtained by density gradient centrifugation. The highest density is Hepatocyte, HSC, Kupffer cells and LSEC, which are obtained by the respective surface characteristic molecules using magnetic beads or flow cytometry sorting. After the cells are collected, they were immediately lysed with TRIZO, mRNA is extracted, and cDNA is obtained by reverse transcription. Finally, the expression of ECMlis detected by RT-PCR. GAPDH is an internal reference.

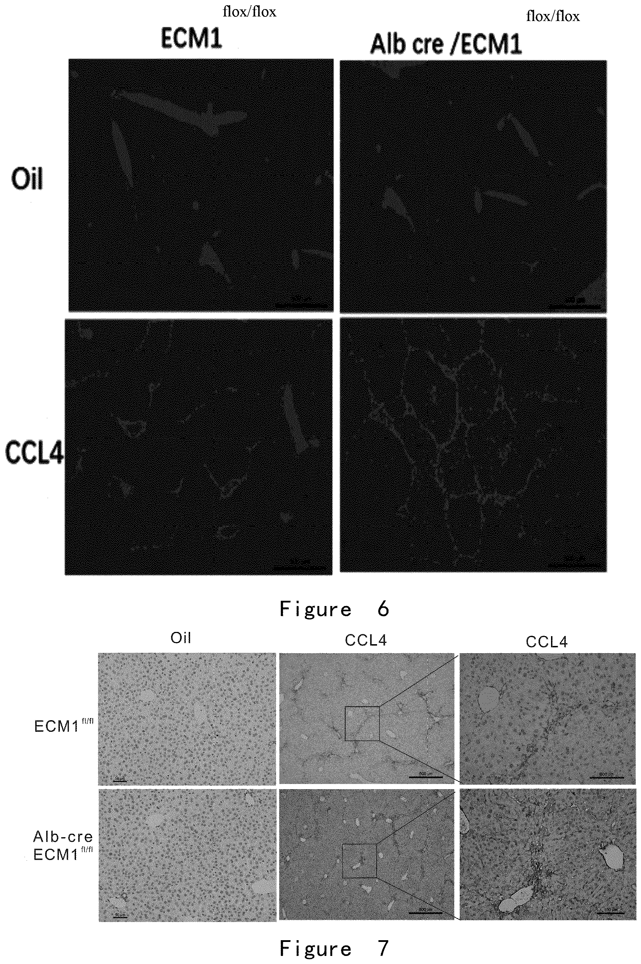

Figure 6 shows that hepatocyte-specific knockout of ECM1 gene promotes liver fibrosis. Wherein

ECM1 wild-type mice (ECM1 ^{flox/flox}) and ECM1 hepatocyte-specific knockout mice (Alb-cre/ECM1 flox/flox) are injected with CCL4 lul/g body weight twice a week for the continuous injection for 4 weeks (CCL4 group), at the same time a group of olive oil injections as a control (Oil group). The mice are sacrificed 2 days after the last injection of CCL4, and taking the liver for fixation, dehydration, embedding, and sectioning, and analyzed by Sirius red collagen staining.

Figure 7 shows that hepatocyte-specific knockout of ECM1 gene promotes stellate cell activation. Wherein

ECM1 wild-type mice (ECM1 flox/flox) and ECM1 hepatocyte-specific knockout mice (Alb-cre/ECM1 flox/flox) are injected with CCL4 lul/g body weight twice a week for the continuous injection for 4 weeks (CCL4 group), at the same time a group of olive oil injections as a control (Oil group). The mice are sacrificed 2 days after the last injection of CCL4, and taking the liver for fixation, dehydration, embedding, and sectioning, and analyzed by a-SMA immunohistochemical staining.

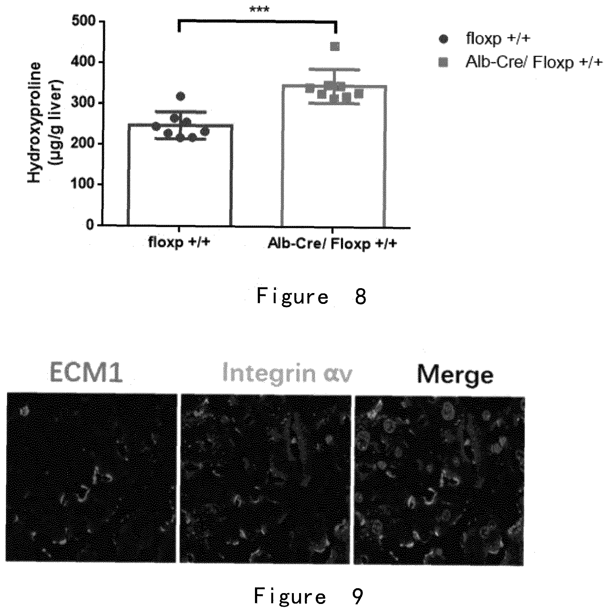

Figure 8 shows that hepatocyte-specific knockout of ECM1 gene promotes liver fibrosis. Wherein

ECM1 wild-type mice (ECM1 ^{flox/flox}) and ECM1 hepatocyte-specific knockout mice (Alb-cre/ECM1 ^{flox/flox}) are injected with CCL4 lul/g body weight twice a week for the continuous injection for 4 weeks (CCL4 group), at the same time a group of olive oil injections as a control (Oil group). The mice are sacrificed 2 days after the last injection of CCL4, and the liver is taken for quantitative analysis of hydroxyproline.

Figure 9 shows the interaction of ECM1 protein with integrin α v.

Wherein wild-type mouse (WT) liver is fixed with PFA, dehydrated, embedded, frozen sectioned, stained with anti-mouse ECM1 antibody and anti-integrin α v, subjected to counterstaining by DAPI, mounted, and photographed. Taking pictures with a laser confocal fluorescence microscope.

Figure 10 shows that ECM1 protein inhibits TGF-β 1 activation and mouse HSC activation. Wherein

A: Isolating a primary stellate cell (HSC) from the liver of wild-type mice (WT) and co-culturing with a NIH-3T3 cell containing the TGF- β 1 activity reporter system. Recombinant mouse ECM1 protein (50 µ g/ml), cRGD (10 µ g/ml) or irrelevant IgG protein (50 µ g/ml) is added to the culture solution as a negative control (NC). After culturing for 16 hours, the cell is lysed and the luciferase activity in the lysate is detected.

B: Isolating a primary stellate cell (HSC) from the liver of wild-type mice (WT) and culturing them in vitro for 2 weeks. Recombinant mouse ECM1 protein (50 µ g/ml), cRGD (10 µ g/ml) or irrelevant IgG protein (50 µ g/ml) is added to the culture solution as a negative control (NC). Changing the fresh culture solution every 3 days. Two weeks later, it is lysed with TRIZO, mRNA is extracted, and cDNA is obtained by reverse transcription. Finally, the expression of related genes is detected by RT-PCR.

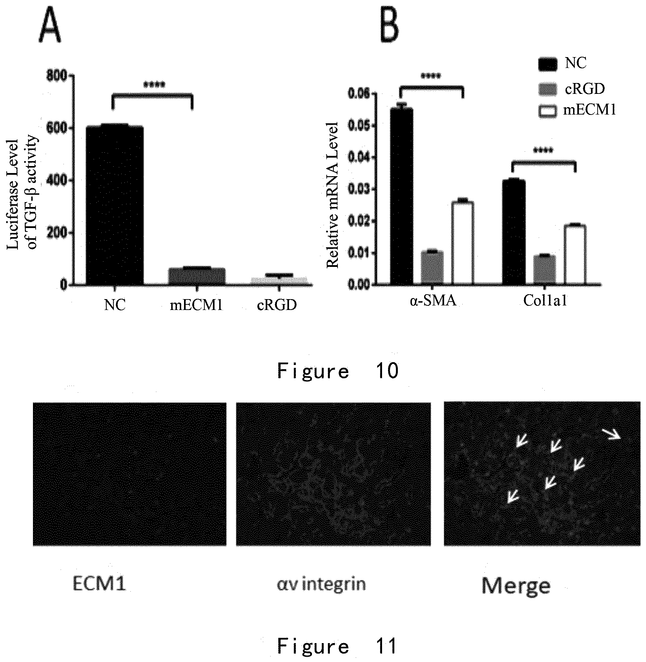

Figure 11 shows the interaction between ECM1 protein and integrin α v in human liver. Wherein healthy human liver tissues are fixed with PFA, dehydrated, embedded, paraffin sectioned, stained with anti-mouse ECM1 antibody and anti-integrin α v, subjected to counterstaining by DAPI, mounted, and photographed. Taking pictures with a laser confocal fluorescence microscope.

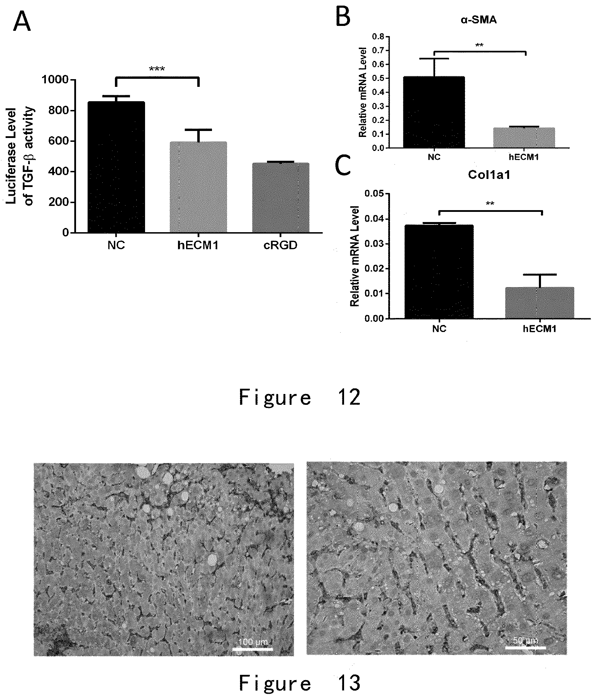

Figure 12 shows that ECM1 protein inhibits the activation of TGF- β and the activation of human HSC. Wherein:
A: Human stellate cells (LX-2) are co-cultured with NIH-3T3 cells containing TGF- β 1 activity reporter system. Recombinant human ECM1 protein (50 µ g/ml), cRGD (10 µ g/ml) or irrelevant IgG protein (50 µ g/ml) is added to the culture solution as a negative control (NC). After culturing for 16 hours, the cells are lysed and the luciferase activity in the lysate is detected.
B and C: Culturing human stellate cells (LX-2) for 2 weeks. Recombinant human ECM1 protein (50 µ g/ml), cRGD (10 µ g/ml) or irrelevant IgG protein (50 µ g/ml) is added to the culture solution as a negative control (NC). Changing the fresh culture solution every 3 days. Two weeks later, it is lysed with TRIZO, mRNA is extracted, and cDNA is obtained by reverse transcription. Finally, the expression of related genes is detected by RT-PCR.

Figure 13 shows that ECM1 protein in human liver is expressed in the extracellular matrix of hepatic sinusoid. Wherein after PFA fixation of healthy human liver tissue, dehydration, embedding, paraffin sectioning, using anti-human ECM1 antibody primary antibody, then incubating with HRP-labeled anti-mouse secondary antibody, finally subjected to DAB color development, hematoxylin counterstaining the nucleus, and mounting the slide, taking pictures.

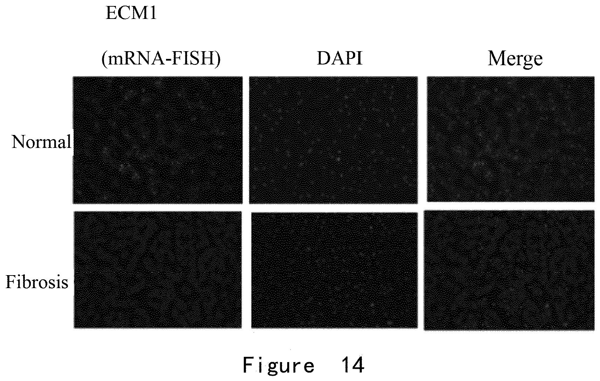

Figure 14 shows that ECM1 protein in human liver is mainly expressed by hepatocytes. Wherein healthy human liver tissues are fixed with PFA, dehydrated, embedded, and paraffin-sectioned and then hybridized with human ECM1 gene probes with red fluorescent markers. The slides are mounted after counterstaining with DAPI. Taking pictures with a laser confocal fluorescence microscope.

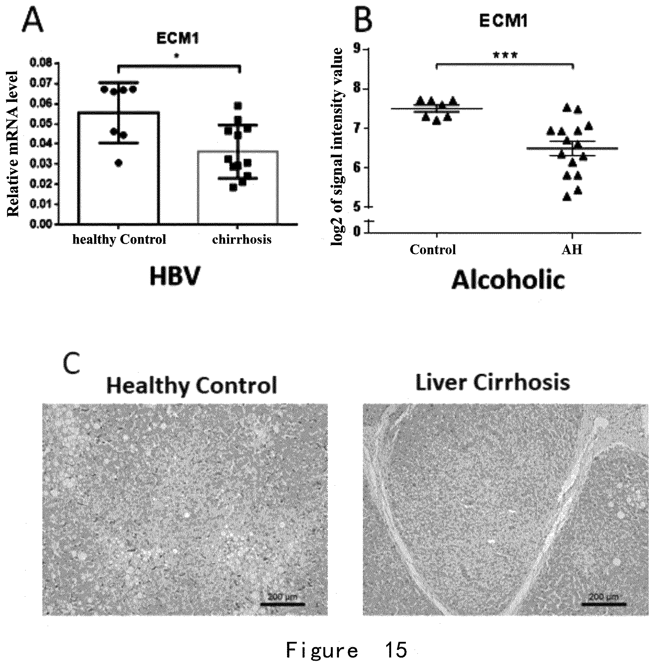

Figure 15 shows the decreased expression of ECM1 in patients with liver cirrhosis. Wherein A: The liver tissues of patients with cirrhosis and healthy people caused by HBV infection are lysed with TRIZO, mRNA is extracted, and cDNA is obtained by reverse transcription. Finally, the expression of ECM1 is detected by RT-PCR. GAPDH is an internal reference.

B: The liver tissues of patients with cirrhosis caused by alcoholic hepatitis and healthy people are lysed with TRIZOL, mRNA is extracted, and cDNA is obtained by reverse transcription. Finally, the expression of ECM1 is detected by gene chip.

C: After the liver tissues of healthy people and patients with liver cirrhosis caused by HBV infection are fixed with PFA, dehydrated, embedded, and paraffin sectioned, using anti-human ECM1 antibody primary antibody, and then incubating with HRP-labeled anti-mouse secondary antibody, and finally subjected to DAB color development, hematoxylin counterstaining the nucleus, and mounting the slide, taking pictures.

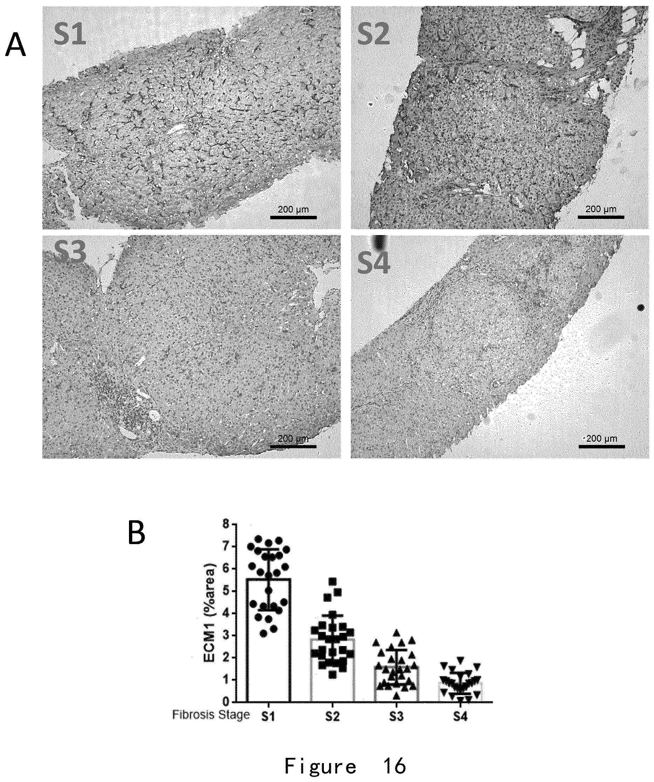

Figure 16 shows that the amount of ECM1 protein is negatively correlated with the degree of liver fibrosis. Wherein A: Taking liver puncture samples from patients with METAVIR staging (S1-S4) of different liver fibrosis. After PFA fixation, dehydration, embedding, and paraffin sectioning, using anti-human ECM1 antibody primary antibody, and then incubating HRP-labeled anti-mouse secondary antibody, finally subjected to DAB color development, hematoxylin counterstaining the nucleus, mounting the slide, and taking pictures.

B: The IHC staining results of the ECM1 protein of the liver puncture samples are counted and plotted for the area of positive staining area using ImageJ software.

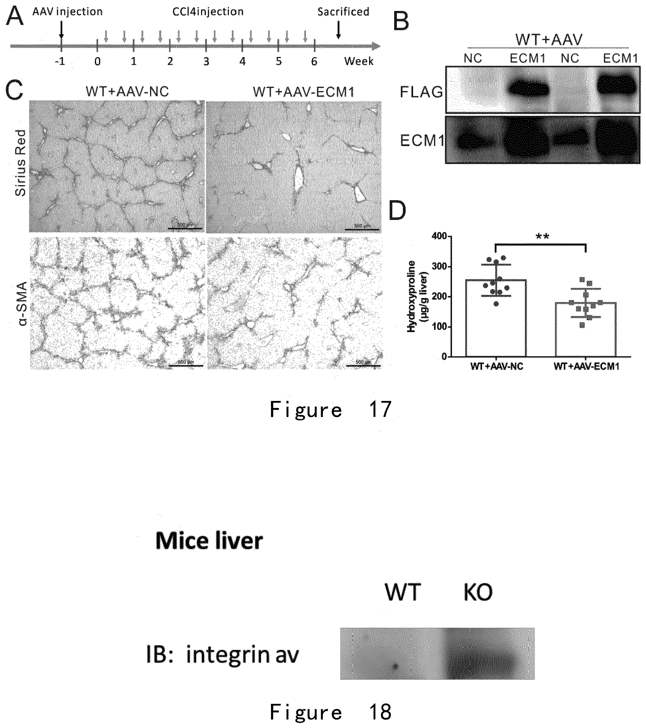

Figure 17 shows that adeno-associated virus-mediated ECM1 protein expression can treat liver fibrosis. Wherein

A: WT mice are divided into 2 groups, 10 mice in each group. Injecting 1×10¹¹ of adeno-associated virus type 2/8 (AAV-ECM1) expressing ECM1 gene or a control virus (AAV-NC) that does not express genes through tail vein, respectively. One week after virus injection, weekly intraperitoneal injection of CC14 for modeling. Six weeks after the injection, the mice are sacrificed and their livers are fixed, dehydrated, embedded, and sectioned. H&E; Masson staining and a-SMA immunohistochemical staining are performed respectively.

B: After 6 weeks of injection, the mice are sacrificed, and 500 mg of liver is collected and lysed in 1 ml of RIPA lysis buffer, and then ground and lysed. After quantification with BCA, uniformly diluted with RIPA lysis buffer to 2ug/ml, adding Loading Buffer to heat denaturation. When loading the sample, each sample is 20ug/lane. Using anti-mouse ECM1 and flag antibody for the detection.

C: The mice are sacrificed and their livers are fixed, dehydrated, embedded, and sectioned. The Sirius red staining and a-SMA immunohistochemical staining are performed respectively.

D: The mice are sacrificed and the liver is lysed. The samples are processed and tested according to the hydroxyproline detection kit. Finally, the value is read at the OD550 wavelength with a spectrophotometer.

Figure 18 shows the increased expression of integrin α v in the liver of ECM1 knockout mice.

### Detailed Description

After extensive and in-depth research, the present inventors have unexpectedly discovered for the first time that the ECM1 gene, or the protein thereof, or the promoter thereof can significantly (i) prevent and/or treat liver cirrhosis-related diseases; and/or (ii) maintain the homeostasis of the liver. In addition, it is also unexpectedly discovered that the ECM1 gene, or the protein thereof, or the promoter thereof can also significantly (i) inhibit the occurrence of liver fibrosis-related diseases; and/or (ii) inhibit the activation of stellate cells (HSC). In addition, the present invention has also unexpectedly discovered that when the expression level of integrin-related genes in blood and/or liver tissue is basically unchanged or up-regulated, ECM1 gene, or the protein thereof, or the promoter thereof can be administered to treat liver fibrosis-related diseases. On this basis, the present inventor has completed the present invention.

As used herein, the structural formula of CWHM 12 is

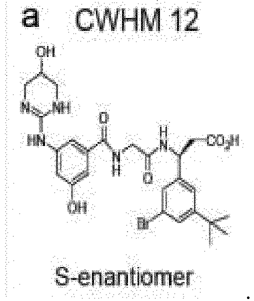

As used herein, the structural formula of c8 (α_{γ}β₁ integrin inhibitor c8) is

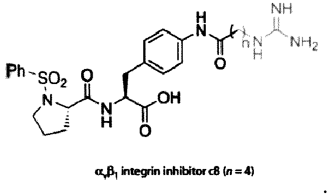

As used herein, the structural formula of PF-573228 is

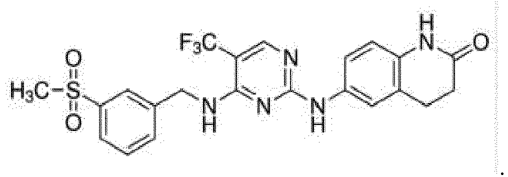

As used herein, the structural formula of EMD527040 is

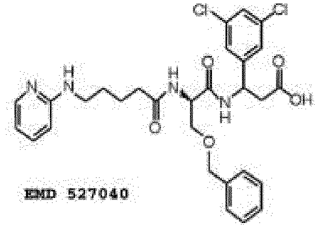

As used herein, the term "the expression level of integrin-related genes in the individual's blood and/or liver tissue is equivalent to a predetermined standard" means that the expression level of integrin-related genes in the individual's blood and/or liver tissue is close to the predetermined standard , or making a certain degree of fluctuation (such as up and down 1%-5%) based on a predetermined standard.

### ECM1 protein and an encoding nucleic acid thereof

As used herein, the terms "ECM1 protein" and "extracellular matrix protein 1" can be used interchangeably.

The present invention relates to an ECM1 protein and a variant thereof. In a preferred embodiment of the present invention, the amino acid sequence of the ECM1 protein is as shown in SEQ ID NO.: 1 or 3. The ECM1 protein or its promoter of the present invention can (i) prevent and/or treat liver cirrhosis-related diseases; and/or (ii) maintain the homeostasis of the liver.

The present invention also includes a polypeptide or protein with the same or similar function that has 50% or more (preferably 60% or more, 70% or more, 80% or more, more preferably 90% or more, more preferably 95% or more, most preferably 98% or more, such as 99%) homology with the sequence as shown in SEQ ID NO. 1 or 3 of the present invention.

Among them, SEQ ID NO.: 1 is a murine ECM1 protein; SEQ ID NO.: 3 is a human ECM1 protein.

The "same or similar function" mainly refers to: "(i) prevention and/or treatment of liver cirrhosis-related diseases; and/or (ii) maintenance of liver homeostasis".

The protein of the present invention can be a recombinant protein, a natural protein, or a synthetic protein. The protein of the present invention can be a natural purified product, or a chemically synthesized product, or produced from a prokaryotic or eukaryotic host (for example, bacteria, yeast, higher plants, insect and mammalian cells) using recombinant technology. Depending on the host used in the recombinant production protocol, the protein of the present invention may be glycosylated or non-glycosylated. The protein of the present invention may also include or not include the initial methionine residue.

The present invention also includes ECM1 protein fragments and analogs having ECM1 protein activity. As used herein, the terms "fragment" and "analog" refer to a protein that substantially maintains the same biological function or activity as the natural ECM1 protein of the present invention.

The mutant protein fragment, derivative or analogue of the present invention may be (i) a mutant protein in which one or more conservative or non-conservative amino acid residues (preferably conservative amino acid residues) are substituted, and such substituted amino acid residues may or may not be encoded by the genetic code, or (ii) a mutant protein with substitution groups in one or more amino acid residues, or (iii) a mutant protein formed by the fusion of a mature mutant protein with another compound (for example, compounds that extend the half-life of mutant proteins, such as polyethylene glycol), or (iv) a mutant protein formed by fusing an additional amino acid sequence to this mutant protein sequence (such as the leader sequence or secretory sequence or the sequence used to purify the mutant protein or the proprotein sequence, or the fusion protein formed with the antigen IgG fragment). According to the teachings herein, these fragments, derivatives and analogs fall within the scope of those skilled in the art. In the present invention, conservatively substituted amino acids are preferably generated by amino acid substitutions according to Table I.

**Table I**

| Initial residues | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also includes that polypeptides or proteins with the same or similar functions that have 50% or more (preferably 60% or more, 70% or more, 80% or more, more preferably 90% or more, more preferably 95% or more, most preferably 98% or more, such as 99%) homology with the natural ECM1 protein of the present invention. The protein variant can be a derivative sequence obtained by several (usually 1-60, preferably 1-30, more preferably 1-20, most preferably 1-10) substitutions, deletions or additions of at least one amino acid, and one or several (usually within 20, preferably within 10, more preferably within 5) amino acids can be added at the C-terminus and/or N-terminus. For example, in the protein, substitution with amino acids with similar or close properties usually does not change the function of the protein. Adding one or several amino acids to the C-terminal and/or \terminal usually does not change the function of the protein. The present invention includes that the difference between the natural ECM1 protein analog and the natural ECM1 protein can be the difference in the amino acid sequence, the difference in the modified form that does not affect the sequence, or both. Analogs of these proteins include natural or induced genetic variants. Induced variants can be obtained through various techniques, such as random mutagenesis through radiation or exposure to mutagens, site-directed mutagenesis or other known molecular biology technology. Analogs also include analogs having residues different from natural L-amino acids (such as D-amino acids), and analogs having non-naturally occurring or synthetic amino acids (such as β, γ-amino acids). It should be understood that the protein of the present invention is not limited to the representative proteins exemplified above.

Modified (usually without changing the primary structure) forms include: chemically derived forms of proteins *in vivo* or *in vitro,* such as acetylation or carboxylation. Modifications also include glycosylation, such as those that undergo glycosylation modifications during protein synthesis and processing. This modification can be accomplished by exposing the protein to an enzyme that performs glycosylation (such as a mammalian glycosylase or deglycosylase). Modified forms also include sequences with phosphorylated amino acid residues (such as phosphotyrosine, phosphoserine, phosphothreonine). In addition, the mutant protein of the present invention can also be modified. Modified (usually not changing the primary structure) forms include: chemically derived forms of mutant proteins *in vivo* or *in vitro,* such as acetylation or carboxylation. Modifications also include glycosylation, such as those produced by glycosylation modification during the synthesis and processing of mutant proteins or further processing steps. This modification can be accomplished by exposing the mutant protein to an enzyme that performs glycosylation (such as a mammalian glycosylase or deglycosylase). Modified forms also include sequences with phosphorylated amino acid residues (such as phosphotyrosine, phosphoserine, phosphothreonine). It also includes mutant proteins that have been modified to increase their resistance to proteolysis or optimize their solubility.

The present invention also provides a polynucleotide sequence encoding ECM1 protein. The polynucleotide of the present invention may be in the form of DNA or RNA. DNA forms include: DNA, genomic DNA or synthetic DNA. DNA can be single-stranded or double-stranded. A polynucleotide encoding a mature polypeptide includes: a coding sequence that only encodes a mature polypeptide; a coding sequence of a mature polypeptide and various additional coding sequences; a coding sequence (and an optional additional coding sequence) and a non-coding sequence of a mature polypeptide. The term "polynucleotide encoding a polypeptide" may include a polynucleotide encoding the polypeptide, or a polynucleotide that also includes additional coding and/or non-coding sequences. The present invention also relates to variants of the above-mentioned polynucleotides, which encode fragments, analogs and derivatives of polypeptides having the same amino acid sequence as the present invention. The variants of this polynucleotide can be naturally occurring allelic variants or non-naturally occurring variants. These nucleotide variants include substitution variants, deletion variants and insertion variants. As known in the art, an allelic variant is an alternative form of a polynucleotide. It may be a substitution, deletion or insertion of one or more nucleotides, but it will not substantially change the function of the encoded polypeptide.

In a preferred embodiment of the present invention, the DNA sequence encoding the human ECM1 protein encodes the ECM1 protein as shown in SEQ ID NO.: 1 or 3, and the polynucleotide sequence encoding the ECM1 protein is shown in SEQ ID NO.: 2 or 4.

Among them, SEQ ID NO.: 2 is a nucleotide sequence of the murine ECM1 gene; SEQ ID NO.: 4 is a nucleotide sequence of the human ECM1 gene.

According to the nucleotide sequence as described herein, those skilled in the art can conveniently use various known methods to prepare the encoding nucleic acid of the present invention. These methods include, but are not limited to: PCR, DNA artificial synthesis, etc., for specific methods, please refer to J. Sambrook, "Molecular Cloning Experiment Guide." As an embodiment of the present invention, the coding nucleic acid sequence of the present invention can be constructed by a method of synthesizing the nucleotide sequence in segments and then performing overlap extension PCR.

The present invention also relates to polynucleotides that hybridize with the aforementioned sequences and have at least 50%, preferably at least 70%, more preferably at least 80% identity between the two sequences. The present invention particularly relates to polynucleotides that can hybridize with the polynucleotide of the present invention under strict conditions (or stringent conditions). In the present invention, "strict conditions" refer to: (1) hybridization and elution at lower ionic strength and higher temperature, such as 0.2 × SSC, 0.1% SDS, 60° C; or (2) denaturant added during hybridization, such as 50% (v/v) formamide, 0.1% calf serum/0.1% Ficoll, 42°C, etc.; or (3) hybridization occurs only when the identity between the two sequences is at least 90% or more, and more preferably 95% or more.

The proteins and polynucleotides of the present invention are preferably provided in an isolated form, and more preferably, are purified to homogeneity.

The full-length sequence of the polynucleotide of the present invention can usually be obtained by PCR amplification method, recombination method or artificial synthesis method. For the PCR amplification method, primers can be designed according to the relevant nucleotide sequence disclosed in the present invention, especially the open reading frame sequence, and a commercially available cDNA library or a cDNA library prepared by a conventional method known to those skilled in the art is used as a template to amplify the relevant sequence. When the sequence is long, it is often necessary to perform two or more times PCR amplifications, and then the amplified fragments are spliced together in the correct order.

Once the relevant sequence is obtained, the recombination method can be used to obtain the relevant sequence in large quantities. This is usually done by cloning it into a vector, then transferring it into a cell, and then isolating the relevant sequence from the proliferated host cell by conventional methods.

In addition, artificial synthesis methods can also be used to synthesize related sequences, especially when the fragment length is short. Usually, by first synthesizing multiple small fragments, and then ligating to obtain fragments with very long sequences.

At present, the DNA sequence encoding the protein (or a fragment or a derivative thereof) of the present invention can be obtained completely through chemical synthesis. The DNA sequence can then be introduced into various existing DNA molecules (or such as vectors) and cells known in the art. In addition, mutations can also be introduced into the protein sequence of the present invention through chemical synthesis.

The method of using PCR technology to amplify DNA/RNA is preferably used to obtain the polynucleotide of the present invention. Especially when it is difficult to obtain full-length cDNA from the library, the RACE method (RACE-rapid amplification of cDNA ends) can be preferably used. The primers used for PCR can be appropriately selected according to the sequence information of the present invention disclosed herein and can be synthesized by conventional methods. The amplified DNA/RNA fragments can be separated and purified by conventional methods such as gel electrophoresis.

### ECM1 promoter

In the present invention, the ECM1 promoter includes substances that can increase the activity and/or content of *ECM1* gene or protein thereof *in vivo* or *in vitro.*

In the present invention, the ECM1 promoter also includes substances that promote the formation of a complex between ECM1 protein and integrin αv.

Wherein, the expression level of ECM1 can be increased by the following methods: the tissue itself secretes a large amount of ECM1 protein or artificially overexpresses ECM1 protein, or artificially delivers ECM1 protein (for example, using viral vectors, such as adeno-associated virus vectors) or ECM1 promoters.

In the present invention, the ECM1 promoter is not particularly limited, as long as it can promote the expression of ECM1 or enhance the activity of ECM1 protein, it is within the protection scope of the present invention.

In a preferred embodiment, the ECM1 promoter includes a small molecule compound.

### Compound pharmaceutical composition and kit

The present invention provides a compound pharmaceutical composition containing active ingredients (a) *ECM1* gene, or a protein thereof or a promoter thereof; and (b) a pharmaceutically acceptable carrier. Such carriers include (but are not limited to): saline, buffer, glucose, water, glycerol, ethanol, powder, and combinations thereof. The pharmaceutical preparation should match the mode of administration. The pharmaceutical composition of the present invention can be prepared in the form of injections, for example, it is prepared by conventional methods with physiological saline or an aqueous solution containing glucose and other adjuvants. Pharmaceutical compositions such as tablets and capsules can be prepared by conventional methods. Pharmaceutical compositions such as injections, solutions, tablets and capsules should be manufactured under aseptic conditions. The pharmaceutical combination of the present invention can also be made into powder for inhalation. The dosage forms of the pharmaceutical composition of the present invention are injections, oral preparations (tablets, capsules, oral liquids), transdermal agents, and sustained-release agents. The amount of active ingredient administered is a therapeutically effective amount. The pharmaceutical preparation of the present invention can also be made into a sustained-release preparation. The pharmaceutical composition of the present invention is preferably an injection preparation. In addition, the pharmaceutical composition of the present invention can also be used together with other therapeutic agents. Moreover, the pharmaceutical composition of the present invention may further include additional components selected from the group consisting of: components for (a) prevention and/or treatment of liver fibrosis-related diseases; and/or (b) maintenance of liver homeostasis.

The effective amount of the active ingredient of the present invention can vary with the mode of administration and the severity of the disease to be treated. The selection of the preferred effective amount can be determined by a person of ordinary skill in the art according to various factors (for example, through clinical trials). The factors include, but are not limited to: the pharmacokinetic parameters of the active ingredients such as bioavailability, metabolism, half-life, etc.; the severity of the disease to be treated by the patient, the patient's weight, the patient's immune status, and route of administration, etc.. Generally, when the active ingredient of the present invention is administered at a dose of about 0.00001 mg-50 mg/kg animal body weight (preferably, 0.0001 mg-10 mg/kg animal body weight), satisfactory effects can be obtained. For example, by the urgent request of the treatment condition, several divided doses can be administered every day, or the dose can be reduced proportionally.

The pharmaceutically acceptable carriers of the present invention include (but are not limited to): water, saline, liposomes, lipids, proteins, protein-antibody conjugates, peptides, cellulose, nanogels, and the combinations thereof. The choice of carrier should match the mode of administration, which are well known to those of ordinary skill in the art.

The present invention also provides a kit that can be used to (a) prevent and/or treat liver fibrosis-related diseases; and/or (b) maintain liver homeostasis, the kit containing:
(i) a first container, and an active ingredient (a) ECM1 gene, or a protein thereof or a promoter thereof, or a medicine containing the active ingredient (a) in the first container; and
(ii) instructions, the instructions for administering an active ingredient (a) so as to (i) prevent and/or treat liver fibrosis-related diseases; and/or (ii) maintain liver homeostasis.

The pharmaceutical composition and kit of the present invention are suitable for (i) preventing and/or treating liver fibrosis-related diseases; and/or (ii) maintaining liver homeostasis.

The preparation of the present invention can be taken three times a day to once every ten days, or once every ten days in a sustained-release manner. The preferred way is to take it once a day, this is because it is convenient for the patient to persist, thereby significantly improving the patient's compliance with medication.

When taking, in most cases, the total daily dose should be lower than (or a few cases equal to or slightly greater than) the daily dose of each single drug. Of course, the effective dose of the active ingredient used can vary depending on the mode of administration and the severity of the disease to be treated.

### Treatment method

The present invention also provides a method for using the two active ingredients of the present invention or corresponding drugs to (i) prevent and/or treat liver fibrosis-related diseases; and/or (ii) maintain liver homeostasis, which includes administering an effective amount of the active ingredient (a) ECM1 gene, or a protein thereof or a promoter thereof to a mammal, or administering a pharmaceutical composition containing the active ingredient (a).

When the two active ingredients of the present invention are used for the above purposes, they can be mixed with one or more pharmaceutically acceptable carriers or excipients, such as solvents, diluents, etc., and can be administered orally in the following forms: tablets, pills, capsules, dispersible powders, granules or suspensions (containing, for example, about 0.05-5% suspending agent), syrups (containing, for example, about 10-50% sugar), and elixirs (containing about 20-50% ethanol), or in the form of a sterile injectable solution or suspension (containing about 0.05-5% suspending agent in an isotonic medium) for parenteral administration. For example, these pharmaceutical preparations may contain about 0.01-99%, more preferably about 0.1%-90% by weight of the active ingredient mixed with the carrier.

The two active ingredients or pharmaceutical compositions of the present invention can be administered by conventional routes, including (but are not limited to): intramuscular, intraperitoneal, intravenous, subcutaneous, intradermal, oral, intratumoral or topical administration . Preferred routes of administration include oral administration, intramuscular administration or intravenous administration.

From the standpoint of ease of administration, the preferred pharmaceutical composition is a liquid composition, especially an injection.

In addition, the two active ingredients or drugs of the present invention can also be used in combination with other components or drugs (such as CWHM 12, c8, PF-573228, EMD527040, etc.) for (a) preventing and/or treating liver fibrosis-related diseases; and/or (b) maintaining liver homeostasis.

### The main advantages of the present invention include:

(1) The present invention has found for the first time that the ECM1 gene, or a protein thereof or a promoter thereof can significantly (a) prevent and/or treat liver fibrosis-related diseases; and/or (b) maintain liver homeostasis.
(2) The present invention has found for the first time that the ECM1 gene, or a protein thereof or a promoter thereof can also significantly (i) inhibit the occurrence of liver fibrosis-related diseases; and/or (ii) inhibit the activation of stellate cells (HSC).
(3) The present invention has discovered for the first time that ECM1 is abundantly expressed in the extracellular matrix of the liver.
(4) The present invention uses the mouse model of knocking out the ECM1 gene in different cells for the first time to prove that the specificity in the hepatocytes (Hepatocyte) significantly reduces the content of ECM1 protein in the liver, and promotes the occurrence of liver fibrosis faster, indicating that the ECM1 protein secreted by hepatocytes plays an important role in maintaining liver homeostasis and inhibiting the occurrence of liver fibrosis. In the pathological state of the liver, the function of liver cells is damaged, and the ability of hepatocytes to produce ECM1 protein is reduced, which promotes the development of liver fibrosis. This result shows that ECM1 protein has an important function of maintaining liver homeostasis in the liver. Down-regulation of ECM1 expression will promote the acceleration of liver fibrosis development.
(5) The present invention has found for the first time that ECM1 protein forms a complex by interacting with integrin αv molecules (Integrin αv), thereby inhibiting the activation of HSC and the production of collagen. Both the administration of ECM1 protein in vitro and the re-expression of ECM1 gene in vivo can both inhibit the activation of HSC and the progression of liver fibrosis, proving that ECM1 is a new molecule with an important role in the treatment of liver fibrosis.
(6) The present invention has found for the first time that the expression of ECM1 in the liver will be down-regulated when normal mouse liver disease occurs and fibrosis continues to progress.
(7) The present invention has found for the first time that the ECM1 protein in human liver is also mainly synthesized and secreted by hepatocytes, and the synthesis of ECM1 protein is reduced after hepatocytes injury, thereby promoting the occurrence of liver fibrosis.
(8) The present invention has found for the first time that ECM1 protein is an important component of liver extracellular matrix. It maintains the homeostasis of liver extracellular matrix and the normal physiological functions of the liver by interacting with integrin αv molecules, and plays an important role in inhibiting liver fibrosis. Since ECM1 gene expression declines in the process of liver fibrosis, it becomes a potentially important therapeutic target.
(9) The present invention has found for the first time that the promoter of ECM1 gene or a protein thereof can promote the formation of complex between ECM1 protein and integrin αv molecule (Integrin αv).
(10) The present invention has discovered for the first time that when the expression level of integrin-related genes in blood and/or liver tissue is unchanged or up-regulated, ECM1 gene, or a protein thereof, or a promoter thereof can be administered to treat liver fibrosis-related diseases.

The present invention will be further explained below in conjunction with specific embodiments. It should be understood that these embodiments are only used to illustrate the present invention and not to limit the scope of the present invention. The experimental methods without specific conditions in the following examples are usually in accordance with conventional conditions such as Sambrook et al., Molecular Cloning: Laboratory Manual (New York: Cold Spring Harbor Laboratory Press, 1989), or in accordance with the conditions recommended by the manufacturer. Unless otherwise stated, percentages and parts are calculated by weight.

Unless otherwise specified, all materials and reagents used in the examples are commercially available products.

### Example 1 ECM1 is abundantly expressed in the extracellular matrix of the liver

ECM1 is a very important extracellular matrix protein, but no article has done a detailed study on its expression in adult mice. In order to further understand the relationship between the expression of ECM1 and the occurrence of liver fibrosis, first, the expression of ECM1 in mice was studied in detail. Different tissues from the same mouse were taken, and after lysis of RIPA, WB is used to observe that ECM1 is mainly expressed in the liver in the parenchymal organs of mice (Figure 1A). It is suggested that ECM1 is a constitutively expressed protein in the liver and plays an important role in the liver. This may be one of the reasons why very obvious liver lesions can be observed in ECM1 whole body knockout mice.

In order to further understand the expression of ECM1 in the liver, frozen sections of mouse liver were made and observed by in situ fluorescent staining of ECM1 in mouse liver. It can be seen from the staining results that the extracellular matrix is enriched in the liver hepatic sinusoid space, and there is a strong staining signal in the crosstalk of different cells in the liver (Figure 1B), indicating that ECM1 is mainly enriched in the extracellular matrix of hepatic sinusoid in the liver, and is an important component of the extracellular matrix of the liver.

### Example 2 The expression of ECM1 decreased during the modelling of liver fibrosis

In order to further understand the relationship between the expression of ECM1 and the occurrence of liver diseases, the changes in the expression of ECM1 were observed in three different liver fibrosis models.

First, CCL4 was administered to normal mice at a dose of 1ul/g body weight twice a week. After 4 weeks, mouse livers were taken for comparison and observation with the control group. It can be seen that after 4 weeks of CCL4 modeling, when the mice begin to show obvious fibrosis, the mRNA expression of ECM1 in the liver is significantly down-regulated (Figure 2A). At the same time, the results of WB also show that the amount of ECM1 protein in the liver has also been reduced by more than half (Figure 2B).

Non-alcoholic fatty liver (NASH) is also a very serious liver disease in the liver field with a large increase in the number of cases. It is one of the major causes of liver fibrosis. So I also want to see how ECM1 changes in this disease. A non-alcoholic fatty liver mouse model method was used: normal mice were fed with methionine/choline deficient feed for 8 weeks, and the mouse liver would show a phenotype similar to human non-alcoholic fatty liver. After 8 weeks of feeding the methionine/choline deficiency feed, comparing the mouse liver with the control group, it can be observed and found that the expression of mRNA and protein of ECM1 in the liver is also significantly down-regulated (Figure 2, C and D).

Lesions in the biliary tract in the liver cause bile to accumulate in the liver, causing chronic liver damage, which is also one of the important causes of liver fibrosis in human liver diseases. Ligation of the bile ducts of normal mice (BDL model) can artificially cause the phenotype of bile to accumulate in the liver, which can be used to simulate liver fibrosis caused by human cholestasis. The livers of the mice were collected on the 3rd and 9th days after the bile duct ligation of the mice, and compared with the mice of the control group. The results show that with the appearance of liver damage and fibrosis caused by cholestasis, the expression of ECM1 mRNA in the liver is continuously down-regulated with the aggravation of the disease (Figure 2E), and the expression of ECM1 protein is also significantly downregulated (Figure 2 F).

### Example 3 ECM1 protein secreted by hepatocytes maintains liver homeostasis and inhibits the occurrence of fibrosis

The main cells in the liver are composed of hepatic parenchymal cells (Hepatocyte), stellate cells (HSC), liver macrophages (Kupffer cells) and liver sinusoidal endothelial cells (LSEC). In addition, some bone marrow-derived immune cells such as monocytes, granulocytes, and T cells infiltrated in the liver. These cells, especially immune cells derived from bone marrow, have different numbers and physiological functions in different disease states of the liver.

The extracellular matrix of the liver is an important structural skeleton of the liver, and it is also a necessary way for different cells in the liver to signal exchange(crosstalk). The extracellular matrix of the liver plays an important role in regulating the functions of various cells in the liver. At the same time, different cells in the liver are constantly dynamically adjusting the composition of the extracellular matrix of the liver.

As an important component of the extracellular matrix of the liver, ECM1 protein is also regulated by different cells in the liver. In order to better study the role of ECM1 protein in liver fibrosis, it is necessary to determine the main source cells of ECM1 protein in the liver at first.

### 3.1 Hepatocytes are the main source of ECM1 protein in the liver

After in situ perfusion with collagenase, the parenchymal cells and non-parenchymal cells can be preliminarily separated by density gradient centrifugation. Parenchymal cells are mainly hepatic parenchymal cells (Hepatocyte) . Non-parenchymal cells suspension only need to be composed of stellate cells (HSC), liver macrophages (Kupffer cells) and liver sinusoidal endothelial cells (LSEC). These cells can be easily separated by centrifugation with different density gradients, but the purity is not enough. Therefore, in order to obtain higher purity cells (>99%), first, using magnetic beads or flow cytometry to obtain HSC, Kupffer cells and liver sinusoidal endothelial cells through their respective surface characteristic molecules, and then performing gene expression analysis.

The results of ECM1 gene expression analysis show that among different cells in the liver, hepatic parenchymal cells (Hepatocyte) have the highest expression of ECM1 (Figure 3A). Considering that >80% of the cells in the liver are hepatic parenchymal cells (Hepatocyte) , hepatic parenchymal cells (Hepatocyte) can be considered to contribute most of the ECM1 protein in the extracellular matrix of the liver.

At the same time, the expression of ECM1 gene in different cells under the condition of liver fibrosis was analyzed. The results show that in CCL4-induced liver fibrosis, the amount of ECM1 gene expressed by hepatic parenchymal cells (Hepatocyte) is significantly down-regulated, while the amount of ECM1 gene expression in other cells does not change much (Figure 3B).

Therefore, it can be considered that hepatic parenchymal cells (Hepatocyte) contribute most of the ECM1 protein in the extracellular matrix of the liver, and when liver fibrosis occurs, as most of the hepatic parenchymal cells (Hepatocyte) are destroyed or their status changes, the expression of ECM1 genes in hepatic parenchymal cells (Hepatocyte) is significantly down-regulated, and finally the amount of ECM1 protein in the extracellular matrix of the liver is reduced.

### 3.2 Hepatocyte specific knockout of ECM1 gene promotes the progress of liver fibrosis

After confirming that hepatic parenchymal cells (Hepatocyte) are the main source of liver ECM1 protein, ECM1 ^{flox/flox} mice (loxP-flanked ("floxed")) were constructed for conditional knockout experiments of ECM1 gene in different cells. Serum albumin (alb) is a protein mainly secreted by hepatic parenchymal cells (Hepatocyte) . Using the promoter of the Alb gene to express the cre enzyme can specifically express the cre enzyme in the hepatic parenchymal cells.

First, ROSA mTmG mice were used to verify the effectiveness and specificity of gene knockout in the liver of Alb-cre mice. In ROSA mTmG mice, only the red fluorescent protein (membrane-targeted tandem dimer Tomato, mT) is expressed on the surface of the cell membrane, and green fluorescent protein (membrane-targeted green fluorescent protein (mG)) is not transcribed because it has a stop codon in front of it. In cells with cre enzyme expression, the red fluorescent protein (mT) gene and the stop codon at the front end of the green fluorescent protein (mG) are cut, and the green fluorescent protein (mG) will replace the red fluorescent protein (mT) and expressed on the cell membrane surface, changing the cell from red to green (Figure 4A). The livers of ROSA mTmG mice and Alb-cre /ROSA mTmG mice were fixed, dehydrated, frozen sectioned and stained with DAPI to label the nucleus. After taking pictures, it can be seen that the liver cell membranes in the liver of ROSA mTmG mice are labeled with red fluorescent protein. In the liver of Alb-cre /ROSA mTmG mice, the surface of all hepatocytes is transformed into green fluorescent protein. This result shows that Alb-cre mice can effectively and specifically knock out the ECM1 gene of hepatocytes in Folxp mice (Figure 4B).

After mating Alb-cre mice with ECM1 ^{flox/flox} mice, homozygous ECM1 hepatic parenchymal cells -specific knockout mice (Alb-cre/ECM1 ^{flox/flox}) were obtained. Firstly, different cells in the liver were sorted, mRNA was extracted, and cDNA was obtained by reverse transcription. Finally, the expression of ECM1 was detected by RT-PCR to confirm that the conditional knockout of ECM1 mediated by Alb-cre only occurs in hepatic parenchymal cells. The ECM1 gene expression in the liver of ECM1 ^{flox/flox} mice is normal (Figure 5). Therefore, ECM1 liver parenchymal cell-specific knockout mice (Alb-cre/ ECM1 ^{flox/flox}) can be used in subsequent liver fibrosis modeling experiments to compare the effects of ECM1 protein produced by hepatocytes on liver fibrosis.

ECM1 wild-type mice (ECM1 flox/flox) and ECM1 hepatic parenchymal cells -specific knockout mice (Alb-cre/ECM1 ^{flox/flox}) were injected with CCL4 lul/g body weight twice a week for 4 consecutive weeks (CCL4 Group), at the same time a group of olive oil injections as a control (Oil group). The mice were sacrificed 2 days after the last injection of CCL4, and the liver was fixed, dehydrated, embedded, sectioned, and analyzed by Sirius red collagen staining.

Experimental results show that in ECM1 hepatic parenchymal cells-specific knockout (Alb-cre/ECM1 flox/flox) mice, there is no spontaneous fibrosis like ECM1 systemic knockout (ECM1-KO) mice. However, after CCL4 was injected twice a week for modeling, ECM1 hepatic parenchymal cells-specific knockout (Alb-cre/ECM1 flox/flox) mice show faster liver fibrosis (Figure 6) and earlier appearance of ascites than ECM1 wild-type mice (ECM1 flox/flox). This result indicates that the ECM1 protein expressed in the liver of ECM1 hepatic parenchymal cells -specific knockout (Alb-cre/ECM1 flox/flox) mice is affected, and the reduction of ECM1 protein in the extracellular matrix promotes the occurrence and development of liver fibers.

After immunohistochemical staining of a-SMA on the liver of mice, it is found that in the liver of ECM1 hepatic parenchymal cells-specific knockout (Alb-cre/ECM1 flox/flox) mice, the amount of a-SMA-positive cells is greater than that of ECM1 wild-type mice (ECM1 flox/flox) (Figure 7). This result is consistent with the Sirius red staining result in the liver. Further observation has found that when the wild-type mouse (ECM1 flox/flox) is modeled, the activated stellate cells generally appear in bundles, concentrated in the space of the pseudolobule. The activated stellate cells in the liver of ECM1 hepatic parenchymal cells-specific knockout (Alb-cre/ECM1 flox/flox) mice not only appear in bundles, but have very strong activated stellate cells in all other hepatic sinusoid spaces. This phenomenon is consistent with the stellate cell activation phenotype observed in the liver of ECM1 whole body knockout mice (ECM1-KO). This result indicates that after specific knockout of ECM1 in the hepatic parenchymal cells, the stellate cells in the hepatic sinusoid lack the protection of the ECM1 protein in the extracellular matrix and are more likely to be activated. The activated stellate cells in the hepatic sinusoid produce a large amount of collagen deposited in the hepatic sinusoid, which can rapidly increase the blood pressure of the hepatic portal vein and promote the occurrence of ascites.

Similarly, in order to perform better statistical analysis on the mice in the experimental group, the livers of all experimental mice were taken for quantitative analysis of hydroxyproline. The results show that the ECM1 wild-type mice (ECM1 flox/flox) and ECM1 hepatic parenchymal cells-specific knockout mice (Alb-cre/ECM1 flox/flox) exhibit no significant difference in hydroxyproline content in the control group (Oil) injected with olive oil, but in the CCL4 model group of mice, the hydroxyproline content in the liver of ECM1 hepatic parenchymal cells-specific knockout mice (Alb-cre/ECM1 flox/flox) is significantly greater than that of ECM1 wild-type mice (ECM1 flox/flox) (Figure 8). This result indicates that the degree of fibrosis in the liver of ECM1 hepatic parenchymal cells-specific knockout mice (Alb-cre/ECM1 flox/flox) during CCL4 modeling is stronger than that of ECM1 wild-type mice (ECM1 flox/flox).

These results indicate that the ECM1 hepatic parenchymal cells-specific knockout mice (Alb-cre/ECM1 flox/flox) after CCL4 modelling will develop faster fibrosis progression than ECM1 wild-type mice (ECM1 flox/flox) . It is proved that the ECM1 protein secreted by hepatic parenchymal cells (Hepatocyte) plays an important role in maintaining liver homeostasis and inhibiting the occurrence of liver fibrosis.

### 3.3 ECM1 protein inhibits the activation of TGFβ1 and the activation of HSC by interacting with integrin αv

Research on ECM1 whole body knockout mice (ECM1-KO) and hepatic parenchymal cells (Hepatocyte) conditional knockout mice (Alb-cre/ ECM1^{flox/flox}) has shown that the knockout of ECM1 gene does not have a significant effect on liver cell damage or inflammation like other classic fibrosis models. ECM1 whole body knockout mice (ECM1-KO) and hepatic parenchymal cells (Hepatocyte) conditional knockout mice (Alb-cre/ ECM1 flox/flox) both have shown that the stellate cells (HSC) in the hepatic sinusoid are activated in situ and transformed into activated fibroblasts. In the process of stellate cell (HSC) activation, the most important and necessary factor is the stimulation of TGF-β1. Stellate cells (HSC) must be stimulated by TGF-β1 to be activated and transformed into activated fibroblasts. In this process, if the function of TGF-β1 is inhibited, the activation of stellate cells (HSC) will also be inhibited..

TGF-β1 can be synthesized by a variety of cells in the liver. But synthetic TGF-β1 is secreted out of the cell in an inactive form at the beginning, and is called Latent TGF-β1. Latent TGF-β1 binds to LAP (latency-associated peptide) via LTBP1 (latent TGF-β1 binding protein 1) to form LLC (large latent complex), which is stored in the extracellular matrix. Latent TGF-β1 needs to be cleaved by specific factors outside the cell and separated from the LAP before it can become active TGF-β1, which binds to the TGF-β1 receptor and activates downstream signaling pathways. Integrins are mainly constitutively expressed on the surface of many kinds of cells. They are a family of heterodimeric receptor molecules composed of α subunits and β subunits. So far, a total of 24 subunits have been found in the integrin family, including 18 α subunits and 6 β subunits. Among the integrin family molecules, there are five integrin molecules containing αv subunits (αvβ1, αvβ3, αvβ5, αvβ6, and αvβ8). These five integrin molecules containing αv subunits can bind to the RGD (arginine-glycine-aspartic acid) tripeptide sequence on the LAP in the TGF-β1 precursor complex and this combination is very important for the maturation and activation of the precursor TGF-β1 under physiological conditions. Subsequent studies have found that αv integrin is necessary in the activation process of TGF-β1. The absence of αv integrin or mutation of the αv integrin binding site on TGF-β1 will make the TGF-β1 precursor in the body unable to mature and activate to produce biologically active TGF-β1, resulting in the loss of the TGF-β1 signaling pathway in the body.

### 3.3.1: ECM1 protein interacts with integrin αV (Integrin αV)

In situ fluorescent staining of mouse liver sections was used to determine whether the ECM1 protein interacted with integrin αV. As shown in Figure 9, the results of immunofluorescence staining show that both ECM1 protein and integrin αV in the liver of mice are strongly positive in the hepatic sinusoid, and red fluorescence can be superimposed with green fluorescence to form a yellow fluorescence signal, indicating that ECM1 protein interacts with integrin αV in mouse liver. As shown in Figure 18, the expression of integrin αV in ECM1 knockout mice is increased significantly, indicating that fibroblasts express a large amount of integrin αV in the process of liver fibrosis.

### 3.3.2 ECM1 protein inhibits the activation of TGF-β1 and the activation of stellate cells HSC in mice

The process of Latent TGF-β1 activation to TGF-β1 is a transient effect. The activated TGF-β1 will bind to the TGF-β1 receptor on the surface of neighboring cells to phosphorylate the downstream SMAD protein, and then activate downstream gene expression. In order to better detect the process of Latent TGF-β1 activation to TGF-β1, we used a co-culture system to detect the TGFβ1 activation process that occurs on the surface of stellate cells HSC. NIH-3T3 cells stably transferred a luciferase reporter gene. There are 4 repeated SMAD binding sites upstream of this reporter gene. Therefore, once Latent TGF-β1 is activated as active TGF-β1, it will bind to the receptor on the surface of NIH-3T3 cells, activate the SMAD signaling pathway downstream of NIH-3T3 cells, and induce the expression of luciferase reporter gene. By detecting the activity of luciferase in NIH-3T3 cells, it can reflect how much Latent TGF-β1 is activated into active TGF-β1 in this co-culture system.

First, isolating primary stellate cells (HSC) from the liver of wild-type mice (WT) and co-culturing with NIH-3T3 cells containing the TGF-β1 activity reporter system. Recombinant mouse ECM1 protein (50µg/ml), cRGD (10µg/ml) or irrelevant IgG protein (50µg/ml) was added to the culture solution as a negative control (NC). After culturing for 16 hours, the cells were lysed and the luciferase activity in the lysate was detected. The experimental results show that the recombinant mouse ECM1 protein can significantly inhibit the activation of Latent TGF-β1 on the surface of stellate cells (HSC) (Figure 10A). In this system, we added a classic integrin αv inhibitor cRGD as a positive control.

The primary stellate cells (HSC) of the mouse liver will self-activate in the process of in vitro culture. This is because of changes in culture conditions, the Latent TGF-β1 secreted by the cells will be activated by the integrin αv on the cell surface to activate stellate cells (HSC). In this process, the addition of integrin αv inhibitor (cRGD) or TGF-β1 neutralizing antibody will inhibit the self-activation of stellate cells (HSC).

Therefore, primary stellate cells (HSC) from the liver of wild-type mice (WT) were isolated and cultured in vitro for 2 weeks. Recombinant mouse ECM1 protein (50µg/ml), cRGD (10µg/ml) or irrelevant IgG protein (50µg/ml) was added to the culture solution as a negative control (NC). Changing the fresh culture solution every 3 days. Two weeks later, it was lysed with TRIZO, mRNA was extracted, and cDNA was obtained by reverse transcription. Finally, the expression of related genes was detected by RT-PCR to determine the degree of activation of primary stellate cells (HSC). The experimental results show that the recombinant mouse ECM1 protein and cRGD significantly inhibit the activation of stellate cells (HSC) (Figure 10B).

These experimental results show that the ECM1 protein has a similar function to cRGD, the Latent TGF-β1 in the extracellular matrix can be inhibited by the interaction with integrin αv. It will be activated by the integrin αv on the cell surface into TGF-β1 to activate the stellate cells (HSC) in the hepatic sinusoid to transform into the activated fibroblasts.

### 3.3.3 ECM1 protein inhibits the activation of human HSC

The LX-2 cell line is a commonly used human stellate cell line, but compared to the primary stellate cells in the liver, the LX-2 cell is already an activated cell line similar to fibroblasts. However, at present, through the detection of a-SMA and collagen and other marker genes in LX-2 cells, LX-2 cells are still widely used for functional detection of genes or small molecules that promote or inhibit the progression of fibrosis.

First, we performed in situ fluorescent staining of healthy human liver slices to determine whether the ECM1 protein interacted with integrin αV. The results of immunofluorescence staining show that both ECM1 protein and integrin αV in human liver are strongly positive in the hepatic sinusoid, and the red fluorescence can be superimposed with the green fluorescence into a yellow fluorescence signal (Figure 11), indicating that ECM1 protein and integrin αV also interact in the human liver.

Then we co-cultured human stellate cells (LX-2) with NIH-3T3 cells containing the TGF-β1 activity reporter system. Recombinant human ECM1 protein (50µg/ml), cRGD (10µg/ml) or irrelevant IgG protein (50µg/ml) was added to the culture solution as a negative control (NC). After culturing for 16 hours, the cells were lysed and the luciferase activity in the lysate was detected. The experimental results show that the recombinant human ECM1 protein can also significantly inhibit the activation of Latent TGF-β1 on the surface of LX-2 cells (Figure 12A).

At the same time, we cultured human stellate cells (LX-2) for 2 weeks. Recombinant human ECM1 protein (50µg/ml), cRGD (10µg/ml) or irrelevant IgG protein (50µg/ml) was added to the culture solution as a negative control (NC). Changing the fresh culture solution every 3 days. Two weeks later, it was lysed with TRIZO, mRNA was extracted, and cDNA was obtained by reverse transcription. Finally, the expression of related genes was detected by RT-PCR. The experimental results show that both recombinant human ECM1 protein and cRGD significantly inhibit the activation of LX-2 cells (Figure 12B).

These experimental results show that human ECM1 protein has a function similar to mouse ECM1 protein, and can inhibit Latent TGF-β1 in the extracellular matrix by interacting with integrin αv, which will be activated by integrin αv on the cell surface into TGF- β1, thereby activating the stellate cells (HSC) in the hepatic sinusoid to transform into activated fibroblasts.

### 3.4: Decreased expression of ECM1 in liver of patients with liver fibrosis

We have observed in mice that the ECM1 protein in the extracellular matrix of the liver is mainly synthesized and secreted by hepatic parenchymal cells (Hepatocyte), and because the liver is stimulated, the hepatic parenchymal cells (Hepatocyte) are damaged, which reduces the synthesis of ECM1 protein, thereby promoting the occurrence of liver fibrosis. In the human body, the liver has always been stimulated by the external environment, such as viral infections, stimulation of alcohol and other hepatotoxic substances, or problems with its own bile metabolism leading to cholestasis and the like.

China is a major liver disease country. About 100 million people are chronically infected by Hepatitis B virus, and 15 million people are chronically infected by Hepatitis C virus. At the same time, a large number of patients suffer from liver fibrosis and liver cirrhosis caused by alcoholic liver and non-alcoholic fatty liver.

Collecting liver fibrosis and liver cirrhosis patient samples, analyzing the expression of ECM1 protein in these samples and its correlation with liver fibrosis.

### 3.4.1: The ECM1 protein in human liver is expressed in the extracellular matrix of hepatic sinusoid

In mice, we have found that ECM1 protein is mainly concentrated in the extracellular matrix of the hepatic sinusoid in the liver, and is an important component of the extracellular matrix of the liver. Therefore, we first studied the distribution of ECM1 protein in the liver of healthy people.

After PFA fixation of healthy human liver tissue, dehydration, embedding, paraffin sectioning, using anti-human ECM1 antibody primary antibody, then incubating with HRP-labeled anti-mouse secondary antibody, finally subjected to DAB color development, hematoxylin counterstaining the nucleus, and mounting the slide , take pictures. The results of the experiment have shown that there are a lot of brown positive staining in the extracellular matrix of the hepatic sinusoid in the liver samples of healthy people (Figure 13), indicating that like mouse ECM1 protein, ECM1 protein in human liver is also mainly enriched in the extracellular matrix of hepatic sinusoid.

### 3.4.2 The ECM1 protein in human liver is mainly expressed by hepatic parenchymal cells

In mice, we have found that most of the ECM1 gene transcription in the liver occurs in the hepatic parenchymal cells. Therefore, first we also studied the ECM1 gene expression in the liver of healthy people.

Because the liver samples of healthy people are very precious, we have no way to get different cells and then analyze them by digesting liver tissue and sorting cells like the mouse experiment. Therefore, we choose to use FISH fluorescent probes to detect the mRNA expression position of ECM1 on the liver slices of healthy people, which can also reflect which cells in the liver express ECM1.

Healthy liver tissues were fixed with PFA, dehydrated, embedded, and paraffin-sectioned and then hybridized with human ECM1 gene probes with red fluorescent markers. Mounting the slides after counterstaining with DAPI. The results of laser confocal fluorescence microscopy show that the red fluorescence signal basically appears in the hepatic parenchymal cells (Hepatocyte) with larger nucleus (Figure 14). Therefore, this shows that it is consistent with the conclusion in mouse liver that most of the gene transcription of ECM1 in human liver occurs in hepatic parenchymal cells (Hepatocyte) .

### 3.4.3: Decreased expression of ECM1 in patients with liver cirrhosis

In mice, we have observed significant down-regulation of ECM1 gene expression in different liver fibrosis models. Therefore, first we also studied the ECM1 gene expression in the liver of healthy people and patients with liver cirrhosis of different etiologies.

Through cooperation with Beijing Friendship Hospital, we obtained liver tissue cDNA from 8 healthy people and 12 liver tissue cDNA from liver cirrhosis patients caused by HBV infection. Detecting the expression level of ECM1 gene in cDNA by RT-PCR, we have found that compared with healthy people, the expression of ECM1 gene in the liver of patients with liver cirrhosis caused by HBV infection is significantly reduced (Figure 15A).

Through cooperation with the University of Heidelberg in Germany, we obtained gene expression microarray data in liver tissues of healthy people and liver tissues of patients with liver cirrhosis caused by alcoholic hepatitis. Extracting the ECM1 gene expression data in it can be found that compared with healthy people, the ECM1 gene expression in the liver of patients with liver cirrhosis caused by alcoholic hepatitis is also significantly reduced (Figure 15B).

Similarly, we have also conducted experimental studies on the expression level of ECM1 protein in the liver of healthy people and patients with liver cirrhosis. Through cooperation with Beijing Friendship Hospital, we obtained liver tissue sections of healthy people and liver tissue sections of patients with liver cirrhosis caused by HBV infection. The paraffin sections were deparaffinized, and after rehydration, the anti-human ECM1 antibody primary antibody was used, and then the HRP-labeled anti-mouse secondary antibody was incubated. Finally, DAB was used for color development, the nucleus was counter-stained with hematoxylin, the slides were mounted, and pictures were taken. The experimental results have found that there are a lot of brown positive stains in the extracellular matrix of the hepatic sinusoid in the liver samples of healthy people, while in patients with liver cirrhosis caused by HBV infection, there is basically no brown positive staining in the liver tissue (Figure 15C). This result shows that it is consistent with the expression of ECM1 mRNA, the expression of ECM1 protein in the liver of patients with liver cirrhosis is also significantly reduced.

The above experimental results show that, consistent with the mouse liver fibrosis model, when human liver cirrhosis occurs, the ability of hepatic parenchymal cells (Hepatocyte) to secrete and produce ECM1 is destroyed.

### 3.5.4: The amount of ECM1 protein is negatively correlated with the degree of liver fibrosis

In liver diseases caused by various etiologies, it takes decades to develop from liver fibrosis to liver cirrhosis. During this process, the hepatic parenchymal cells (Hepatocyte) continue to be damaged, inflammation in the liver is enhanced, activated stellate cells increase, and collagen continues to accumulate. Therefore, we want to know whether the ECM1 protein is gradually reduced as the hepatic parenchymal cells (Hepatocyte) continue to be damaged in the process of the continuous development of liver disease and liver fibrosis. If this is the case, it is possible to treat liver fibrosis by supplementing ECM1 protein or restoring the ECM1 gene expression ability of hepatic parenchymal cells (Hepatocyte) in this process.

Through cooperation with Guangzhou Nanfang Hospital, we obtained liver biopsy puncture sample of patients with different liver fibrosis stages caused by HBV infection (METAVIR score staging, S1 to S4, 20 samples for each stage). After PFA fixation, dehydration, embedding, paraffin sectioning, using anti-human ECM1 antibody primary antibody, then incubating with HRP-labeled anti-mouse secondary antibody, finally subjected to DAB color development, hematoxylin counterstaining cell nucleus, mounting, and taking pictures. The results show that during the continuous development of liver fibrosis, the ECM1 protein content in the hepatic sinusoid is continuously decreasing (Figure 16A). In order to better statistically analyze the changes of ECM1 protein in the patient's liver, we used ImageJ software to calculate and plot the area of the stained positive area for the IHC staining results of the ECM1 protein of the liver puncture samples. The results of statistical analysis also show that during the continuous development of liver fibrosis, the ECM1 protein content in the hepatic sinusoid is decreased gradually (Figure 16B).

These experimental results indicate that in the process of liver disease, the continuous damage of hepatic parenchymal cells ( Hepatocyte ) reduces the expression of ECM1 protein in the hepatic sinusoid, and promotes the development of liver fibrosis.

### Example 4 Adeno-associated virus-mediated ECM1 expression inhibits CCl₄-induced liver fibrosis in mice

The previous study have found that when liver fibrosis occurs in mice, the expression of ECM1 protein in the liver decreases. Therefore, trying to use adeno-associated virus to re-express ECM1 protein in the mouse liver during this process to see if it can inhibit the occurrence of liver fibrosis.

WT mice were divided into 2 groups, 10 mice in each group. 1×1011 adeno-associated virus type 2/8 (AAV-ECM1) expressing ECM1 gene or a control virus (AAV-NC) without expressing genes was injected through the tail vein, respectively. One week after the virus injection, weekly intraperitoneal injection of CCl4 for modeling. Six weeks after the injection, the mice were sacrificed and their livers were fixed, dehydrated, embedded, and sectioned. Sirius Red staining and a-SMA immunohistochemical staining were performed separately (Figure 17A).

It is first discovered that adeno-associated virus can re-express the ECM1 gene in the liver of mice modeled by CCl₄ (Figure 17B).

The results of Sirius red staining and a-SMA immunohistochemical staining show that a large amount of reddish material deposits is appeared in the tissues of mice injected with a control virus that does not express genes group (AAV-NC), and liver fibrosis occurs faster and more severely. However, the liver of mice injected with adeno-associated virus type 2/8 (AAV-ECM1) expressing the ECM1 gene contains less collagen, indicating that the occurrence of fibrosis is inhibited (Figure 17C). The results of the hydroxyproline detection kit show that the hydroxyproline content in the liver of mice injected with the adeno-associated virus type 2/8 (AAV-ECM1) expressing the ECM1 gene is much lower than the (AAV-NC) group of mice (Figure 17D).

These experimental results show that using adeno-associated virus type 2/8 to mediate the re-expression of the ECM1 gene in the liver can inhibit fibrosis in the liver of mice. The above experimental results show that ECM1 protein can also inhibit the activation of stellate cells and the production of liver fibrosis in mice.

### Discussion

In this part of the experimental results, it is found to be consistent with the experimental results observed in mice. In the human liver, the ECM1 protein in the extracellular matrix of the liver is mainly synthesized and secreted by hepatic parenchymal cells (Hepatocyte), and because the hepatic parenchymal cells (Hepatocyte) are damaged after the liver is stimulated, the synthesis of ECM1 protein is reduced, thereby promoting the occurrence of liver fibrosis.

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

## Claims

1. Use of *ECM1* gene, or a protein thereof or a promoter thereof for the preparation of a composition or a preparation for (a) preventing and/or treating a liver fibrosis-related disease; and/or (b) maintaining liver homeostasis.

2. The use of claim 1, wherein the composition or preparation is also used to (i) inhibit the occurrence of a liver fibrosis-related disease; and/or (ii) inhibit the activation of a hepatic stellate cell (HSC).

3. The use of claim 1, wherein the ECM1 promoter is selected from the group consisting of a small molecule compound, a vector expressing ECM1, and a combination thereof.

4. The use of claim 1, wherein the ECM1 protein is selected from the group consisting of:
(A) A polypeptide whose amino acid sequence is shown in SEQ ID NO.: 1 or 3;
(B) a ECM1 protein derivative or an active fragment thereof formed by substitution, deletion or addition of one or several (usually 1-60, preferably 1-30, more preferably 1-20, most preferably 1-10) amino acid residues in the amino acid sequence as shown in SEQ ID NO.: 1 or 3;
(C) compared with the amino acid sequence as shown in SEQ ID NO.: 1 or 3, a ECM1 protein derivative or active fragment thereof with homology ≥90%, preferably ≥95%, more preferably ≥98%, and most preferably ≥99%.

5. The use of claim 1, wherein the ECM1 gene is selected from the group consisting of:
(a) a polynucleotide encoding the polypeptide as shown in SEQ ID NO.: 1 or 3;
(b) a polynucleotide whose sequence is shown in SEQ ID NO.: 2 or 4;
(c) a polynucleotide whose nucleotide sequence has a homology of ≥95% (preferably ≥98%) with the sequence as shown in SEQ ID NO.: 2 or 4 and encoding the polypeptide as shown in SEQ ID NO.: 1 or 3;
(d) a polynucleotide complementary to any of the polynucleotides as described in (a) to (c).

6. A pharmaceutical composition comprising:
(a1) a first active ingredient for preventing and/or treating a liver fibrosis-related disease, the first active ingredient comprises: ECM1 gene, or a protein thereof or a promoter thereof;
(a2) a second active ingredient for preventing and/or treating a liver fibrosis-related disease, the second active ingredient comprises: other drugs for preventing and/or treating a liver fibrosis-related disease; and
(b) a pharmaceutically acceptable carrier.

7. The pharmaceutical composition of claim 6, wherein the other drugs for preventing and/or treating a liver fibrosis-related disease are selected from the group consisting of: CWHM 12, c8, PF-573228, EMD527040, and a combination thereof.

8. A kit comprising:
(i) a first container, and the active ingredient (a1) ECM1 gene, or a protein thereof or a promoter thereof, or a drug containing the active ingredient (a) in the first container; and
(ii) a second container, and the active ingredient (a2) other drugs for preventing and/or treating a liver fibrosis-related disease, or a drug containing the active ingredient (a2) in the second container.

9. A method for inhibiting a stellate cell (HSC) activation, comprising the steps:
in the presence of a ECM1 gene, or a protein thereof or a promoter thereof, culturing a stellate cell, thereby inhibiting the activation of the stellate cell (HSC).

10. A method for screening a potential therapeutic agent for a liver fibrosis-related disease, comprising:
(a) In a test group, in the culture system, in the presence of a test compound, culturing a cell expressing the *ECM1* gene for a period of time T1, and detecting the expression level E1 of the *ECM1* gene in the culture system of the test group;
and in a control group where the test compound is absent and other conditions are the same, detecting the expression level E2 of the *ECM1* gene in the culture system of the control group; and
(b) comparing E1 and E2, if E1 is significantly higher than E2, indicating that the test compound is a potential therapeutic agent for liver fibrosis.

11. A kit comprising:
(a1) a first container, and a therapeutic drug located in the first container, the therapeutic drug comprising a ECM1 gene, or a protein thereof, or a promoter thereof; and
(b1) a second container, and a detection reagent for an integrin-related gene in the second container.

12. A method for determining a therapeutic regimen, comprising:
a) providing a test sample from a subject;
b) detecting the expression level of the integrin-related gene in the test sample;
and
c) determining a therapeutic regimen based on the expression level of the integrin-related gene in the sample.
